# EUROPEAN PATENT SPECIFICATION

(11) **EP 2 349 242 B1**
(45) Date of publication and mention of the grant of the patent: **05.12.2018**
(21) Application number: 09748621.1
(22) Date of filing: 29.10.2009
(51) Int. Cl.: A61K 31/155, A61K 31/5575, C07C 279/14, C07C 291/02, C07C 405/00

(54) **AMINO ACID SALTS OF PROSTAGLANDINS**
AMINOSÄURESALZE VON PROSTAGLANDINEN
SELS D'ACIDES AMINÉS DE PROSTAGLANDINES

(30) Priority: 29.10.2008 US 260534; 29.10.2008 US 260522
(43) Date of publication of application: 03.08.2011
(73) Proprietor: Novaer Holdings, Inc., Bedminster, NJ 07921 (US)
(72) Inventor: DELONG, Mitchell, A., Raleigh NC 27617 (US); STURDIVANT, Jill, Marie, Chapel Hill NC 27516 (US)
(74) Representative: Grünecker Patent- und Rechtsanwälte PartG mbB
(86) International application number: PCT/US2009/062590
(87) International publication number: WO 2010/096123

(56) References cited:
- WO-A2-02/096868
- GB-A- 2 025 413
- US-A- 3 931 282
- US-A- 3 985 791
- US-A- 4 005 133

## Description

The present invention is directed to novel amino acid salts of prostaglandins, to a pharmaceutical composition comprising said salts and to the salts for use in the treatment of certain medical disorders.

Various prostaglandins are useful for the treatment of medical conditions including, for example: ocular disorders, such as glaucoma; skin disorders; circulatory disorders, such as hypertension; gastrointestinal disorders; hair loss; respiratory disorders; fertility control; and bone disorders, such as osteoporosis. Information regarding the biological effects of Prostaglandin F analogs is disclosed in the following references: PCT Publication No. WO 99/12895, 1999; PCT Publication No. WO 99/12896, 1999; PCT Publication No. WO 99/12898; *Abstr.* **1999**, *194116* "Molecular mechanisms of diverse actions of prostanoid receptors", Biochimica et Biophysica Acta, 1259 (1995) 109-120; U.S. Patent No. 3,776,938 issued to Bergstrom, S., and Sjovall, J., December 4, 1973; U.S. Patent No. 3,882,241 issued to Pharriss, G., May 6, 1975; G.B. Patent No. 1,456,512 (1976) issued to Pfizer Inc., Bundy, G.L.; Lincoln, F.H., "Synthesis of 17-Phenyl-18,19,20-trinor prostaglandins I. The PG1 Series", Prostaglandins Vol. 9 (1975) pp. 1-4.; CRC Handbook of Eicosanoids: Prostaglandins and Related Lipids Vol. 1, Chemical and Biochemical Aspects, Parts A & B, A.L. Willis, eds., CRC Press (1987); Liljebris, C.; et. al. "Derivatives of 17-Phenyl-18,19,20-trinorprostaglandin F2α Isopropyl Ester: Potential Antiglaucoma Agents", Journal of Medicinal Chemistry Vol. 38, (1995), pp. 289-304; Collins, P.W.; Djuric, S.W. "Synthesis of Therapeutically Useful Prostaglandin and Prostacyclin Analogs", Chemical Reviews 93 (1993), pp. 1533-1564.

All naturally occurring prostaglandins, including PGF_{2α}, and almost all non-naturally-occurring prostaglandins possess a carboxylic acid moiety at the C₁ position. The carboxylic acid moiety is a site for metabolic degradation by *beta* oxidation, which contributes to the rapid metabolism of the naturally occurring prostaglandins. Attempts to prevent *beta* oxidation by modifying the carboxylic acid moiety at the 1 position as an ester moiety, a sulfonamide moiety, and as a tetrazole are known in the art (See e.g. PCT Publication No. WO 99/12895, 1999; PCT Publication No. WO 99/12896, 1999; PCT Publication No. WO 99/12898). However, such modifications have either resulted in only modest increases in half-life (such as the esters) or resulted in compounds with diminished potency.

Prostaglandin F analogs wherein C₁ itself is replaced with a heteroatom have also been described in the art. For example, PGF analogs containing a sulfonic acid moiety at C₁ (The chemistry of prostaglandins containing the sulfo group. Iguchi, Y.; Kori, S.; Hayashi, M. J. Org. Chem., 40, pp. 521-523 1975) and PGF analogs containing a phosphonic acid moiety at C₁ (The Synthesis of dimethylphosphonoprostaglandin analogs, Kluender, H. C. & Woessner, W. Prostaglandins and Medicine, 2: pp.441-444, 1979) have been disclosed. However, such compounds suffer from significantly diminished potency. However, the potent C₁ carboxylic acids are difficult to purify as they are most often synthesized as oils.

US4005133 (A) discloses the L-arginine salt oif PGF_{2α} and a process for preparing a free-flowing solid form thereof. Further, the preparation and use of this salt in regulation of the estrus of domestic animals is described.

WO02096868 (A2) discloses a new and effective process for the synthesis of 17-phenyl-18,19,20-trinor-PGF_{2α} and its derivatives, including the anti-glaucoma drugs Bimatoprost and Latanoprost.

There is a need for solid forms of prostaglandins, both naturally and non-naturally occurring, for the purposes of purification and as intermediates in synthesis, as well as the direct use of these solids in drug products.

The present invention provides an amino acid prostaglandin free acid salt, wherein the amino acid is arginine; and wherein the prostaglandin free acid is selected from the group consisting of latanoprost free acid and travoprost free acid, and wherein the free acid salt is crystalline.

In another embodiment, the present invention provides a pharmaceutical composition comprising the amino acid prostaglandin free acid salt of the invention and a pharmaceutically acceptable carrier.

In another embodiment, the present invention provides the amino acid prostaglandin free acid salt of the invention for use in improving nasal patency, treating glaucoma or other eye disease, skin disorders, respiratory disorders, circulatory disorders, gastrointestinal disorders, hair loss, fertility control, osteoporosis, or neurogenic bladder.

In another embodiment, the present invention provides the amino acid prostaglandin free acid salt of the invention for use in reducing ocular pressure.

The present specification further discloses the following 61 embodiments.
1. An amino acid salt of a prostaglandin free acid.
2. The salt according to embodiment 1, wherein the salt has a melting point of at least about 35°C.
3. The salt according to embodiment 1, wherein the amino acid is a basic amino acid.
4. The salt according to embodiment 1, wherein the prostaglandin free acid is a FP receptor ligand.
5. The salt according to embodiment 1, wherein the prostaglandin free acid is an EP receptor ligand.
6. The salt according to embodiment 1, wherein the amino acid comprises a guanidine group.
7. The salt according to embodiment 1, wherein the prostaglandin free acid is a compound according to Formula (II): 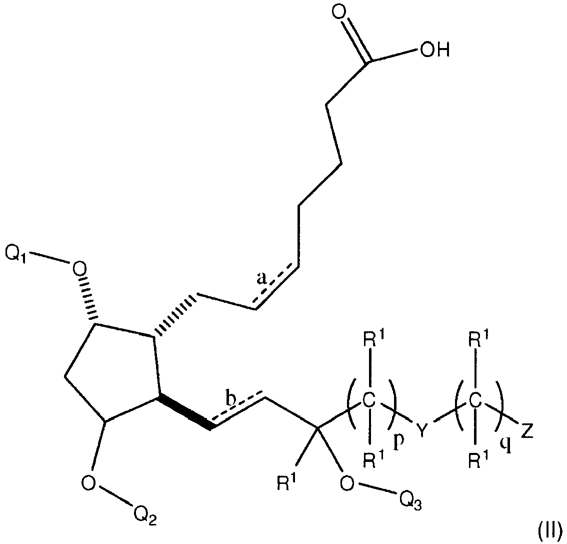 optical isomers, diastereomers, or enantiomers thereof;
   wherein a and b are independently, single, double or triple bonds;
   wherein Q₁, Q₂ and Q₃ are independently selected from hydrogen or alcohol protecting group;
   wherein each R¹ is independently selected from hydrogen or lower alkyl;
   wherein Y is -O-, -S-, -S(O)-, -SO₂-, -C(R²)₂-, -NR¹-, -CR²=CR²-, or -C≡C-;
   wherein Z is selected from hydrogen, carbocycle, aryl, or heteroaryl;
   wherein each R², if present, is independently selected from hydrogen, lower alkyl, alkoxy or hydroxyl; and
   wherein p and q are independently an integer from 0 to 4.
8. The salt according to embodiment 7, wherein Z is bicyclic.
9. The salt according to embodiment 7, wherein Z is substituted.
10. The salt according to embodiment 7, selected from the group consisting of
   7-((1R,2R,3R,5S)-3,5-dihydroxy-2-((S,E)-3-hydroxy-5-phenylpent-1-enyl)cyclopentyl)heptanoic acid, arginine salt: 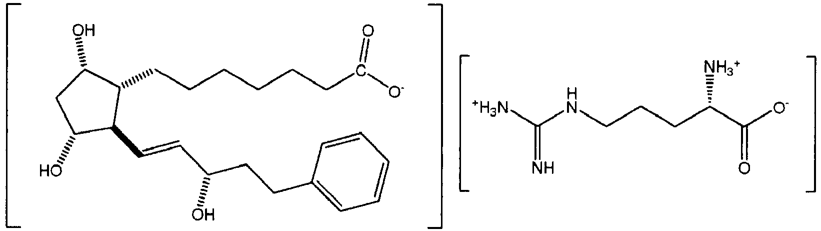 7-((1R,2R,3R,5S)-3,5-dihydroxy-2-((R)-3-hydroxy-4-phenoxybutyl) cyclopentyl)heptanoate, arginine salt: 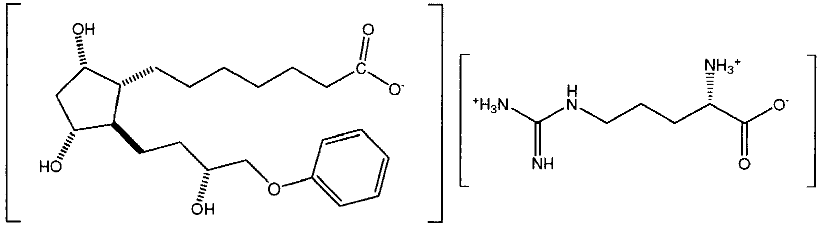 Arginyl (Z)-7-((1R,2R,3R,5S)-3,5-dihydroxy-2-((R,E)-3-hydroxy-4-phenoxybut-1-enyl) cyclopentyl) hept-5-enoate: 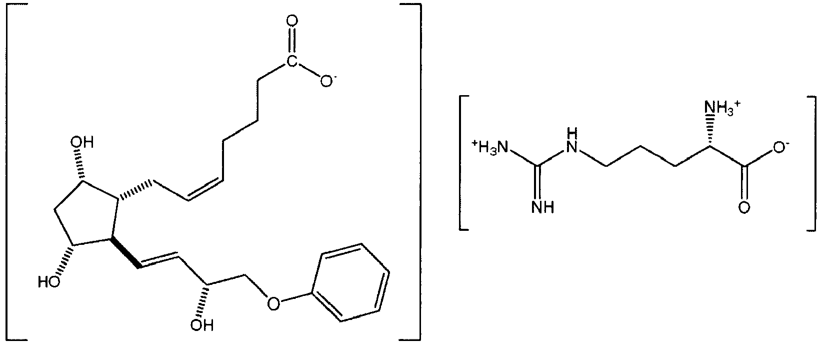 Arginyl (Z)-7-((1R,2R,3R,5S)-2-((R,E)-4-(3-chlorophenoxy)-3-hydroxybut-1-enyl)-3,5-dihydroxy cyclopentyl)hept-5-enoate: 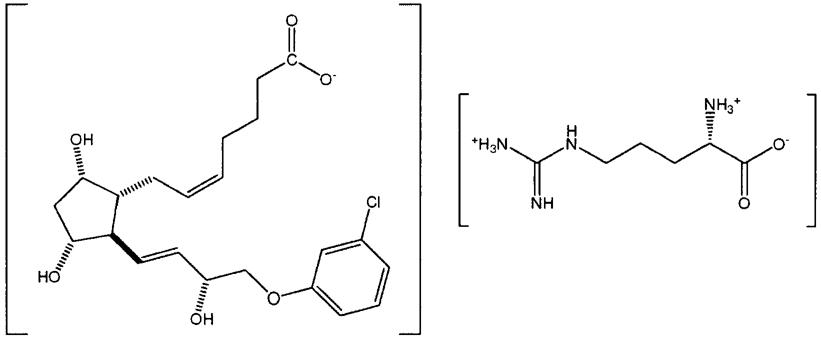 Arginyl (Z)-7-((1R,2R,3R,5S)-3,5-dihydroxy-2-((R,E)-3-hydroxy-4-(3-(trifluoromethyl)phenoxy) but-1-enyl)cyclopentyl)hept-5-enoate 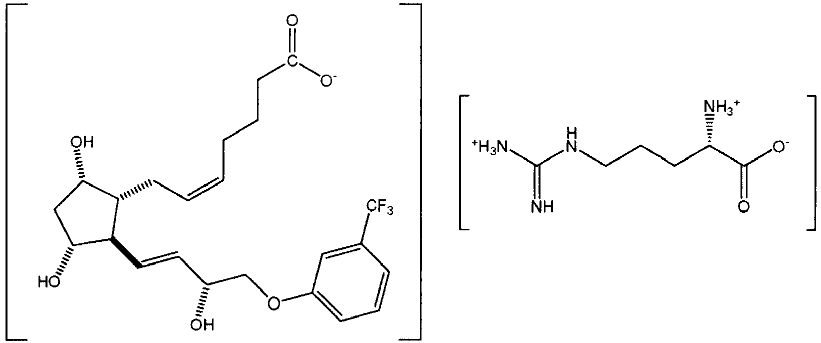 Arginyl (Z)-7-((1R,2R,3R,5S)-2-((R,E)-4-(2-fluorophenoxy)-3-hydroxybut-1-enyl)-3,5-dihydroxycyclopentyl)hept-5-enoate 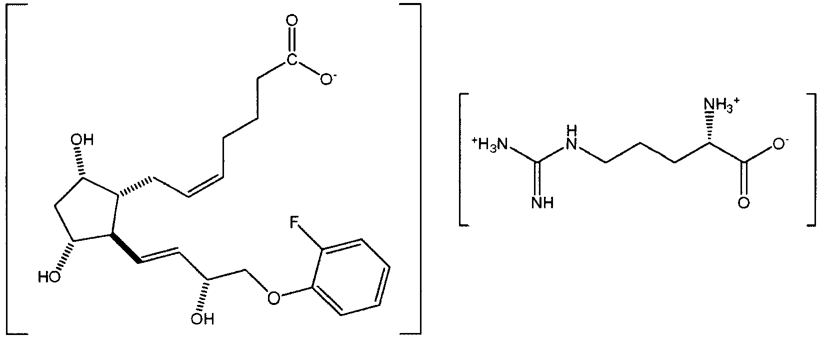 Arginyl (Z)-7-((1R,2R,3R,5S)-2-((R,E)-4-(2-fluorophenylthio)-3-hydroxybut-1-enyl)-3,5-dihydroxycyclopentyl)hept-5-enoate 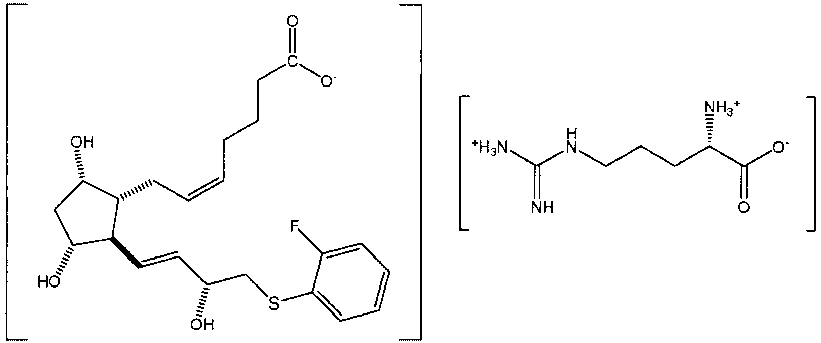 Arginyl 7-((1R,2R,3R,5S)-2-((R)-5-(2-fluorophenyl)-3-hydroxypent-4-ynyl)-3,5-dihydroxycyclopentyl) heptanoate 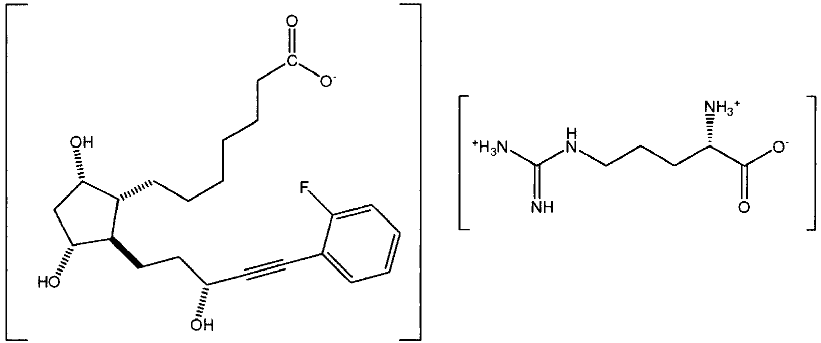 Arginyl 7-((1R,2R,3R,5S)-2-((R)-4-(2-fluorophenylthio)-3-hydroxybutyl)-3,5-dihydroxy cyclopentyl)heptanoate 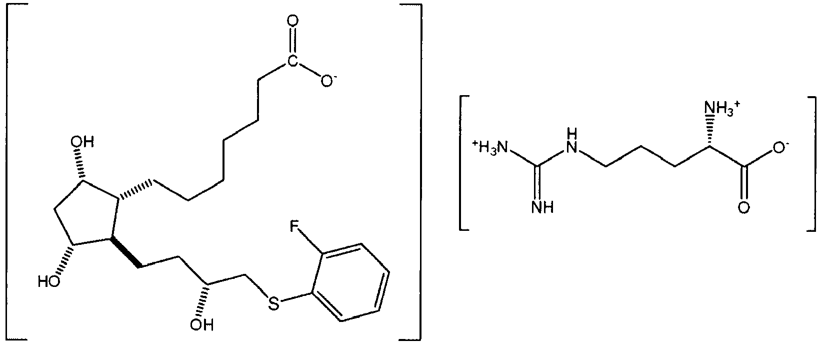 Arginyl (Z)-7-((1R,2R,3R)-3-hydroxy-2-((S,E)-3-hydroxy-5-phenylpent-1-enyl)-5-oxo cyclopentyl)hept-5-enoate 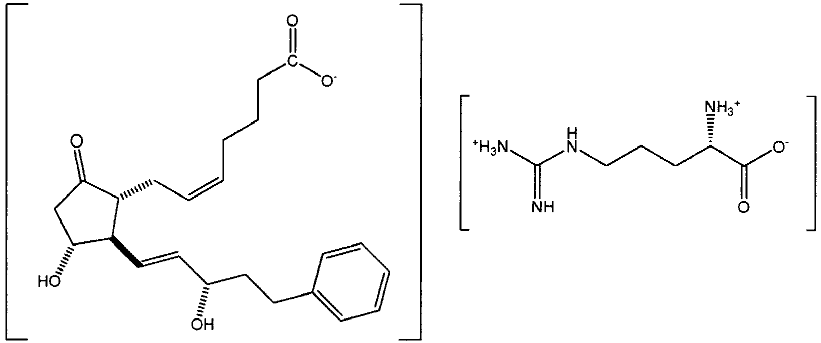 Arginyl 7-((1R,2R,3R)-3-hydroxy-2-((S,E)-3-hydroxy-5-phenylpent-1-enyl)-5-oxocyclopentyl)heptanoate 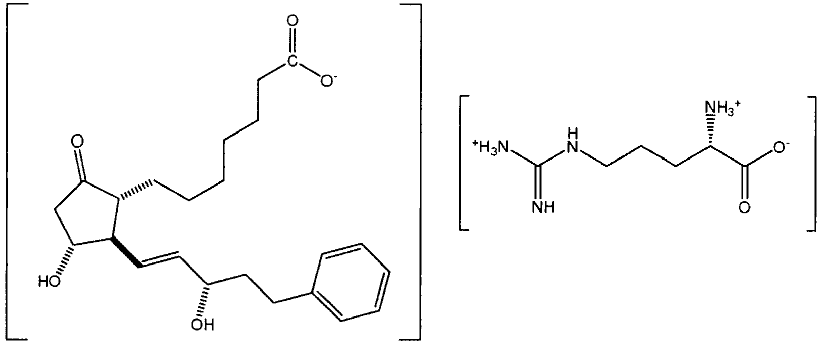 PGE₁, arginine salt 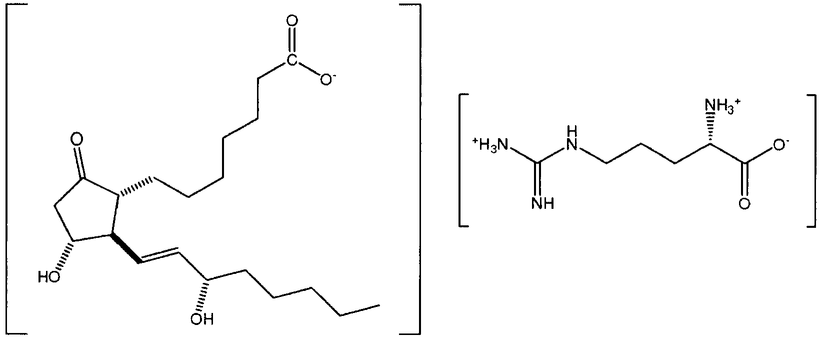 PGE₂, arginine salt 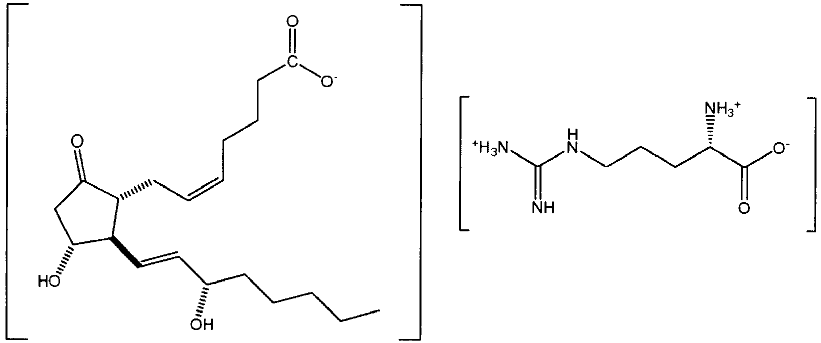 or
   PGD2 arginine salt: 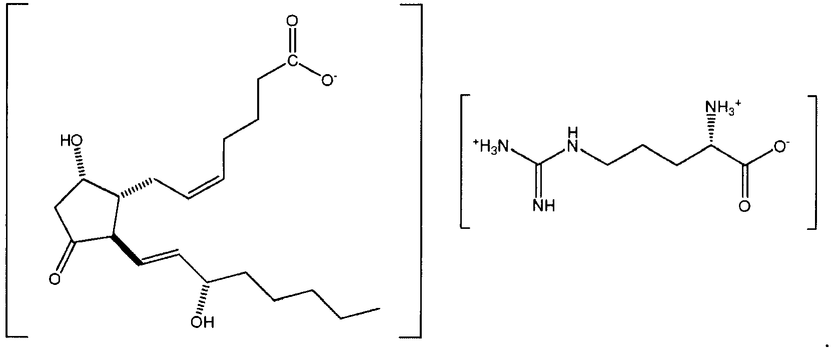
11. The salt according to embodiment 1, wherein the prostaglandin free acid is tafluprost free acid.
12. The salt according to embodiment 1, wherein the prostaglandin free acid is bitamoprost free acid.
13. The salt according to embodiment 1, wherein the prostaglandin free acid is latanoprost free acid.
14. The salt according to embodiment 1, wherein the prostaglandin free acid is fluprostenol free acid.
15. The salt according to embodiment 1, wherein the salt is a product of a reaction between a prostaglandin free acid and an amino acid.
16. A pharmaceutical composition comprising a salt according to embodiment 1 and a pharmaceutically acceptable carrier.
17. A method of making an amino acid salt of a prostaglandin free acid comprising reacting a prostaglandin free acid with a basic amino acid to form an amino acid salt of prostaglandin free acid.
18. A method of making a prostaglandin analog comprising:
   reacting a prostaglandin free acid with a basic amino acid to form a salt; and
   converting the salt to a prostaglandin analog.
19. The method of embodiment 18, wherein the prostaglandin analog is an ester.
20. The method of embodiment 18, wherein the prostaglandin analog is an amide.
21. The method of embodiment 18, wherein the prostaglandin analog binds to the prostaglandin F receptor.
22. The method of embodiment 18, wherein the basic amino acid comprises a guanidino group.
23. A method of making a prostaglandin analog comprising:
   reacting a prostaglandin free acid with a basic amino acid to form a salt;
   neutralizing the salt to recover the prostaglandin free acid; and
   converting the prostaglandin free acid to a prostaglandin analog.
24. The method of embodiment 23, wherein the prostaglandin analog is an ester.
25. The method of embodiment 23, wherein the prostaglandin analog is an amide.
26. The method of embodiment 23, wherein the prostaglandin analog binds to the prostaglandin F receptor.
27. The method of embodiment 23, wherein the basic amino acid comprises a guanidino group.
28. A method of making a prostaglandin comprising
   reacting a prostaglandin free acid with a basic amino acid to form a salt; and
   converting the salt to a prostaglandin.
29. The method of embodiment 28, wherein the prostaglandin binds to the prostaglandin F receptor.
30. The method of embodiment 28, wherein the basic amino acid comprises a guanidino group.
31. A method of treating glaucoma or other eye disease comprising administering to a subject in need thereof an effective amount of a salt according to embodiment 1.
32. A method of improving nasal patency or treating neurogenic bladder comprising administering to a subject in need thereof an effective amount of a salt according to embodiment 1.
33. A method of treating osteoporosis comprising administering to a subject in need thereof an effective amount of a salt according to embodiment 1.
34. A method of treating skin disorders, respiratory disorders, circulatory disorders, gastrointestinal disorders, hair loss or fertility control comprising administering to a subject in need thereof an effective amount of a salt according to embodiment 1.
35. A method of reducing ocular pressure comprising contacting a cell with an amount of a salt according to embodiment 1 effective to reduce ocular pressure.
36. The method of embodiment 35, wherein the cell is in a subject.
37. An amino acid prostaglandin free acid salt, wherein the amino acid is selected from the group consisting of arginine, homoarginine, N(delta)-methyl-L-arginine, L-canavanine, D-canavanine, DL-canavanine, L-α-amino-β-guanidinopropionic acid, γ-guanidinobutyric acid, and citrulene; and wherein the prostaglandin free acid is selected from the group consisting of latanoprost free acid, travoprost free acid, fluprostenol free acid, tafluprost free acid, and bitamoprost free acid.
38. The salt according to embodiment 1, wherein the amino acid is arginine.
39. The salt according to embodiment 2, wherein the prostaglandin free acid is travoprost free acid.
40. The salt according to embodiment 2, wherein the prostaglandin free acid is fluprostenol free acid.
41. The salt according to embodiment 2, wherein the prostaglandin free acid is tafluprost free acid.
42. The salt according to embodiment 2, wherein the prostaglandin free acid is bimatoprost free acid.
43. An amino acid prostaglandin free acid salt, wherein the prostaglandin free acid is latanoprost free acid and the amino acid is arginine.
44. A pharmaceutical composition comprising a salt according to embodiment 1 and a pharmaceutically acceptable carrier.
45. A method of making a prostaglandin free acid salt comprising reacting a mixture comprising a
   prostaglandin free acid selected from the group consisting of latanoprost free acid, travoprost free acid, fluprostenol free acid, tafluprost free acid, and bitamoprost free acid with an amino acid selected from the group consisting of arginine, homoarginine, N(delta)-methyl-L-arginine, L-canavanine, D-canavanine, DL-canavanine, L-α-amino-β-guanidinopropionic acid, γ-guanidinobutyric acid, and citrulene to form a salt.
46. A method of making a prostaglandin analog comprising:
   reacting a prostaglandin free acid selected from the group consisting of latanoprost free acid, travoprost free acid, fluprostenol free acid, tafluprost free acid, and bitamoprost free acid with an amino acid selected from the group consisting of arginine, homoarginine, N(delta)-methyl-L-arginine, L-canavanine, D-canavanine, DL-canavanine, L-α-amino-β-guanidinopropionic acid, γ-guanidinobutyric acid, and citrulene to form a salt; and
   converting the salt to a prostaglandin analog.
47. The method of embodiment 10, wherein the prostaglandin analog is an ester.
48. The method of embodiment 10, wherein the prostaglandin analog is an amide.
49. The method of embodiment 10, wherein the prostaglandin analog binds to the prostaglandin F receptor.
50. A method of making a prostaglandin analog comprising:
   reacting a prostaglandin free acid selected from the group consisting of latanoprost free acid, travoprost free acid, fluprostenol free acid, tafluprost free acid, and bitamoprost free acid with an amino acid selected from the group consisting of arginine, homoarginine, N(delta)-methyl-L-arginine, L-canavanine, D-canavanine, DL-canavanine, L-α-amino-β-guanidinopropionic acid, γ-guanidinobutyric acid, and citrulene to form a salt;
   neutralizing the salt to recover the prostaglandin free acid; and
   converting the prostaglandin free acid to a prostaglandin analog.
51. The method of embodiment 14, wherein the prostaglandin analog is an ester.
52. The method of embodiment 14, wherein the prostaglandin analog is an amide.
53. The method of embodiment 14, wherein the prostaglandin analog binds to the prostaglandin F receptor.
54. A method of making a prostaglandin comprising
   reacting a prostaglandin free acid selected from the group consisting of latanoprost free acid, travoprost free acid, fluprostenol free acid, tafluprost free acid, and bitamoprost free acid with an amino acid selected from the group consisting of arginine, homoarginine, N(delta)-methyl-L-arginine, L-canavanine, D-canavanine, DL-canavanine, L-α-amino-β-guanidinopropionic acid, γ-guanidinobutyric acid, and citrulene to form a salt; and
   converting the salt to a prostaglandin.
55. The method of embodiment 18, wherein the prostaglandin binds to the prostaglandin F receptor.
56. A method of treating glaucoma, or other eye disease comprising administering to a subject in need thereof an effective amount of a salt according to embodiment 1.
57. A method of improving nasal patency or treating neurogenic bladder comprising administering to a subject in need thereof an effective amount of a salt according to embodiment 1.
58. A method of treating osteoporosis comprising administering to a subject in need thereof an effective amount of a salt according to embodiment 1.
59. A method of treating skin disorders, respiratory disorders, circulatory disorders, gastrointestinal disorders, hair loss or fertility control comprising administering to a subject in need thereof an effective amount of a salt according to embodiment 1.
60. A method of reducing ocular pressure comprising contacting a cell with an amount of a salt according to embodiment 1 effective to reduce ocular pressure.
61. The method of embodiment 24, wherein the cell is in a subject.
   Figure 1 is an image of recrystallized Latanoprost-L-Arginine salt **2**, described in Example 88, at 400x magnification.
   Figure 2 is an image of recrystallized Latanoprost-L-Arginine salt **2**, described in Example 88, at 400x magnification.
   Figure 3 is an image of recrystallized Latanoprost-L-Arginine salt **2**, described in Example 88, at 400x magnification.
   Figure 4 is an image of recrystallized Latanoprost-L-Arginine salt **2**, described in Example 88, at 40x magnification.
   Figure 5 is an image of recrystallized Latanoprost-L-Arginine salt **2**, described in Example 88, at 40x magnification.
   Figure 6 is an image of recrystallized Latanoprost-L-Arginine salt **2**, described in Example 88, at 40x magnification.
   Figure 7 is an image of recrystallized Latanoprost-L-Arginine salt **2**, described in Example 88, at 400x magnification.
   Figure 8 is an image of recrystallized Latanoprost-L-Arginine salt **2**, described in Example 88, at 400x magnification.
   Figure 9 is an image of recrystallized Latanoprost-L-Arginine salt **2**, described in Example 88, at 400x magnification.
   Figure 10 is an image of recrystallized Latanoprost-L-Arginine salt **2**, described in Example 88, at 400x magnification.

The present invention is directed to novel amino acid salts of prostaglandin free acids and the present specification further discloses methods of making and using these salts. The amino acid prostaglandin salts are useful in methods of treating eye diseases, such as glaucoma. The amino acid prostaglandin salts of the present invention are also useful in a method of treating bone disorders, such as osteoporosis, treating skin disorders, treating respiratory disorders, treating circulatory disorders such as hypertension, treating gastrointestinal disorders, treating hair loss, fertility control, improving nasal patency or treating neurogenic bladder.

### Definitions and Usage of Terms

"Alcohol protecting group" refers to a protecting group that replaces the active hydrogen of a hydroxyl moiety thus preventing undesired side reaction at the hydroxyl moiety. Use of alcohol protecting groups in organic synthesis is well known in the art. Examples of alcohol protecting groups are found in Chapter 2 of Theodora W. Greene's text entitled: "Protective Groups in Organic Synthesis" (3rd Edition). Suitable alcohol protecting groups include silyl ethers, alkoxymethyl ethers, tetrahydropyranyl, tetrahydrofuranyl, esters, and substituted or unsubstituted benzyl ethers. If more than one alcohol protecting group is present, the alcohol protecting groups may be the same or different. In some embodiments, the alcohol protecting groups may be orthogonal.

"Alkyl" refers to a saturated or unsaturated hydrocarbon chain having 1 to 18 carbon atoms, suitably 1 to 12 carbon atoms, or 1 to 6 carbon atoms, or 1 to 4 carbon atoms. Alkyl gropus may be straight or branched. In some embodiments, branched alkyl groups have one or two branches. Unsaturated alkyl groups have one or more double bonds and/or one or more triple bonds. Suitably, unsaturated alkyl groups have one or two double bonds or one triple bond. Alkyl chains may be unsubstituted or substituted with from 1 to about 4 substituents unless otherwise specified. Suitably, alkyl groups are mono-, di-, or tri-substituted. Suitable alkyl substituents include, but are not limited to, cyano, halo, hydroxy, aryl (e.g., phenyl, tolyl, alkyloxphenyl, alkyloxycarbonylphenyl, halophenyl), heterocyclyl, and heteroaryl.

"Aromatic ring" refers to an aromatic hydrocarbon ring system. Aromatic rings are monocyclic or fused bicyclic ring systems. Monocyclic aromatic rings contain from about 5 to about 10 carbon atoms, suitably from 5 to 7 carbon atoms, or from 5 to 6 carbon atoms in the ring. Bicyclic aromatic rings contain from 8 to 12 carbon atoms, suitably 9 or 10 carbon atoms in the ring. Aromatic rings may be unsubstituted or substituted with from 1 to about 4 substituents on the ring. Suitable aromatic ring substituents include, but are not limited to, halo, cyano, lower alkyl, heteroalkyl, haloalkyl, phenyl, phenoxy or any combination thereof. Suitably, the aromatic ring substituents are lower alkyl, cyano, halo or halo alkyl.

"Basic amino acid" refers to naturally-occurring and non-naturally-occurring alpha, beta and gamma amino acids, including all enantiomers and diastereomers, whether protected or unprotected, provided that the amino acids have a net positive charge at neutral pH. Neutral pH is a pH from about 6.5 to about 7.5. The basic amino acid may be basic when it is the free acid form, or it may only be basic as the ester or amide form of the amino acid. Suitable basic amino acids that are basic in the free acid form include, but are not limited to, arginine, lysine, histidine, L-Arginine, D-Arginine, DL-Arginine, homoarginine, 3- and 4-substituted arginine analogs, N(delta)-methyl-L-arginine (deltaMA), L-canavanine, D-canavanine, DL-canavanine, protected and/or substituted analogs of canavanine, L-α-Amino-β-guanidinopropionic acid, γ-guanidinobutyric acid, citrulline, 3-guanidinopropionic acid, 4-{[amino(imino)methyl]amino}butanoic acid, 6-{[amino(imino)methyl]amino}hexanoic acid, L-2-Amino-3-guanidinopropionic acid, L-Homoarginine, L-Arginine hydroxamate, Agmatine (CAS #: 2482-00-0), and NG-Methyl-L-arginine (CAS #: 53308-83-1). Other amino acids can be made into a basic amino acid if the acid group or groups are suitably protected. Suitable examples of basic amino acids that are esters include L-Arginine ethyl ester. Suitable examples of basic amino acids that are protected as amides include, but are not limited to, L-Argininamide, L-Arginine N-ethylamide, and Arg-beta-Ala hydrochloride, (CAS #: 98957-79-0). In some embodiments, the basic amino acid comprises a guanidino group.

"Carbocycle" refers to a saturated or unsaturated hydrocarbon ring. Carbocycles are not aromatic. Carbocycles are monocyclic, or are fused, spiro, or bridged bicyclic ring systems. Monocyclic carbocycles contain from about 4 to about 10 carbon atoms, suitably from 4 to 7 carbon atoms, or from 5 to 6 carbon atoms in the ring. Bicyclic carbocycles contain from 8 to 12 carbon atoms, suitably from 9 to 10 carbon atoms in the ring. Carbocycles may be unsubstituted or substituted with from 1 to about 4 substituents on the ring. Suitable carbocycle substituents include, but are not limited to, halo, cyano, lower alkyl, heteroalkyl, haloalkyl, phenyl, phenoxy or any combination thereof. Suitably, the carbocycle substituents are halo or haloalkyl. Suitable carbocycles include, but are not limited to, cyclopentyl, cyclohexyl, cyclohexenyl, cycloheptyl, and cyclooctyl.

"Halo" refers to fluoro, chloro, bromo or iodo.

"Haloalkyl" refers to a straight, branched, or cyclic hydrocarbon substituted with one or more halo substituents. Suitably, the haloalkyl is C₁-C₁₂, or C₁-C₆, or C₁-C₃. Suitable halo substituents include fluoro and chloro. One suitable haloalkyl is trifluoromethyl.

"Heteroalkyl" refers to a saturated or unsaturated chain containing carbon and at least one heteroatom, wherein no two heteroatoms are adjacent. Heteroalkyl groups contain from 1 to 18 member atoms (carbon and heteroatoms) in the chain, or 1 to 12 member atoms, or 1 to 6 member atoms, or 1 to 4 member atoms. Heteroalkyl groups may be straight or branched. Suitably, the branched heteroalkyl may have one or two branches. Unsaturated heteroalkyl have one or more double bonds and/or one or more triple bonds. Suitably, heteroalkyl groups have one or two double bonds or one triple bond. Heteroalkyl groups may be unsubstituted or substituted with from 1 to about 4 substituents unless otherwise specified. Suitable heteroalkyl substituents include halo, aryl (e.g., phenyl, tolyl, alkyloxyphenyl, alkyloxycarbonylphenyl, halophenyl), heterocyclyl, heteroaryl. For example, alkyl chains substituted with the following substituents are heteroalkyl: alkoxy (e.g., methoxy, ethoxy, propoxy, butoxy, pentoxy), aryloxy (e.g., phenoxy, chlorophenoxy, tolyloxy, methoxyphenoxy, benzyloxy, alkyloxycarbonylphenoxy, acyloxyphenoxy), acyloxy (e.g., propionyloxy, benzoyloxy, acetoxy), carbamoyloxy, carboxy, mercapto, alkylthio, acylthio, arylthio (e.g., phenylthio, chlorophenylthio, alkylphenylthio, alkoxyphenylthio, benzylthio, alkyloxycarbonylphenylthio), amino (e.g., amino, mono- and di- C₁-C₃ alkanylamino, methylphenylamino, methylbenzylamino, C₁-C₃ alkanylamido, carbamamido, ureido, guanidino).

"Heteroatom" refers to a nitrogen, sulfur, or oxygen atom. Groups containing more than one heteroatom may contain different heteroatoms. As used herein, halogens are not considered heteroatoms.

"Heterocycle" refers to a saturated or unsaturated ring containing carbon and from 1 to about 4 heteroatoms in the ring, wherein no two heteroatoms are adjacent in the ring and no carbon in the ring that has a heteroatom attached to it also has a hydroxyl, amino, or thiol group attached to it. Heterocycles are not aromatic. Heterocycles are monocyclic, or are fused or bridged bicyclic ring systems. Monocyclic heterocycles contain from about 4 to about 10 member atoms (carbon and heteroatoms), suitably from 4 to 7 member atoms, or from 5 to 6 member atoms in the ring. Bicyclic heterocycles contain from 8 to 12 member atoms, suitably 9 or 10 member atoms in the ring. Heterocycles may be unsubstituted or substituted with from 1 to about 4 substituents on the ring. Suitably, the substituents are halo or haloalkyl. Suitable heterocycle substituents include: halo, cyano, lower alkyl, heteroalkyl, haloalkyl, phenyl, phenoxy or any combination thereof. Suitable heterocycles include, but are not limited to, piperzyl, morpholinyl, tetrahydrofuranyl, tetrahydropyranyl and piperdyl.

"Heteroaryl" refers to an aromatic ring system containing carbon and from 1 to about 4 heteroatoms in the ring. Heteroaryls are monocyclic or fused bicyclic ring systems. Monocyclic heteroaryls contain from about 5 to about 10 member atoms (carbon and heteroatoms), or from 5 to 7 member atoms, or from 5 to 6 member atoms in the ring. Bicyclic heteroaryls contain from 8 to 12 member atoms, or 9 or 10 member atoms in the ring. Heteroaryls may be unsubstituted or substituted with from 1 to about 4 substituents on the ring. Suitable heteroaryl substituents include: halo, cyano, lower alkyl, heteroalkyl, haloalkyl, phenyl, phenoxy or any combination thereof. Suitably, the substituents are halo, haloalkyl or phenyl. Suitable heteroaryls include, but are not limited to, benzothienyl, benzofuranyl, thienyl, thiazolo, purinyl, pyrimidyl, pyridyl, and furanyl.

"Lower alkyl" refers to an alkyl chain comprised of 1 to 4 carbon atoms, suitably 1 to 3 carbon atoms or 1 to 2 carbon atoms. Lower alkyl groups may be saturated or unsaturated and substituted or unsubstituted. Lower alkyl groups include, but are not limited to, methyl, ethyl, propyl, isopropyl, butyl, isobutyl and t-butyl.

"Lower heteroalkyl" refers to a heteroalkyl chain comprised of 1 to 4 member atoms. Lower heteroalkyl groups may be saturated or unsaturated and substituted or unsubstituted.

"Member atom" refers to a polyvalent atom (C, O, N, or S atom) in a chain or ring system that continues the chain or ring system. For example, in benzene the six carbon atoms are member atoms and the six hydrogen atoms are not member atoms.

"Phenyl" refers to a six-membered monocyclic aromatic ring which may or may not be substituted with from about 1 to about 4 substituents. The substituents may be substituted at the *ortho*, *meta* or *para* position on the phenyl ring, or any combination thereof. Suitable phenyl substituents include: halo, cyano, lower alkyl, heteroalkyl, haloalkyl, phenyl, phenoxy or any combination thereof.

"Prostaglandin analog" refers to a compound that is designed to bind to a prostaglandin receptor. Prostaglandin analogs include protected prostaglandins, e.g. prostaglandins with esters or amides at the C1 position.

"Prostaglandin free acid" refers to a prostaglandin, prostaglandin analog or prostaglandin intermediate that has a carboxylic acid moiety at the C1 position.

"Prostaglandin intermediate" refers to a compound that is on the synthetic pathway to a compound that, in its active form, stimulates one or more of the prostaglandin receptors. Prostaglandin intermediates may be a protected prostaglandin or prostaglandin analog or they may have parts of their structures not yet attached. An example of a prostaglandin intermediate is a latanoprost free acid containing one or more silyl protecting groups on one or more of their alcohol moieties.

"Prostaglandin F analog" or "PGF analog" or "analog of PGF_{2α}" refers to a compound that is structurally similar to naturally occurring PGF_{2α}.

"Prostaglandin E analog" or "PGE analog" or "analog of PGE₂" or "analog of PGE₁" refers to a compound that is structurally similar to naturally occurring PGE₂.

"Prostaglandin D analog" or "PGD analog" or "analog of PGD₂" refers to a compound that is structurally similar to naturally occurring PGD₂.

"Protected" refers to a chemical structure wherein one or more of the chemically-sensitive groups in the molecule have been modified to reduce its activity and allow for better synthetic techniques to be used. In prostaglandins, typical groups where protection is used are the hydroxy groups and the carboxcyclic acid group. Less commonly protected are the ketones found in PGD and PGE analogues. Protecting groups vary but are generally found in the various editions of "Protecting Groups in Organic Synthesis" by Theadora Green.

"Unprotected" refers to a chemical structure that does not contain any groups that have been added to protect sensitive functional moieties such as hydroxy groups or carboxcylic acid groups.

"Pharmaceutically acceptable carrier" refers to a carrier that is useful for the preparation of a pharmaceutical composition that is: generally compatible with the other ingredients of the composition, not deleterious to the recipient, and neither biologically nor otherwise undesirable.

"A pharmaceutically acceptable carrier" includes both one and more than one carrier. Embodiments include carriers for topical, ocular, parenteral, intravenous, intraperitoneal intramuscular, sublingual, nasal and oral administration. "Pharmaceutically acceptable carrier" also includes agents for preparation of aqueous dispersions and sterile powders for injection or dispersions.

"Excipient" refers to a physiologically compatible additives useful in preparation of a pharmaceutical composition. Examples of pharmaceutically acceptable carriers and excipients can for example be found in Remington Pharmaceutical Science, 16th Ed.

"Therapeutically effective amount" refers to a dosage of the compounds or compositions effective for influencing, increasing, decreasing the acitivty of a receptor, in particular a prostaglandin receptor in a mammal. This term as used herein may also refer to an amount effective at bringing about a desired in vivo effect in a mammal, preferably, a human, such as reduction in intraocular pressure.

"Administering" refers to administration of the compounds as needed to achieve the desired effect.

"Eye disease" includes, glaucoma, allergy, cancers of the eye, neurodegenerative diseases of the eye, and dry eye.

The term "contacting a cell" is used to mean contacting a cell in vitro or in vivo (i.e. within a subject, such as a mammal, including humans, rabbits, cats and dogs).

### Amino Acid Prostaglandin Salts

The present specification discloses novel amino acid salts of prostaglandin free acids suchs as those of Formula I below: 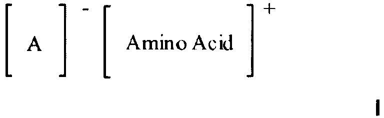 wherein [A]⁻ is an anion of a prostaglandin free acid, and all optical isomers, diastereomers, enantiomers, solvates and hydrates thereof. In one embodiment, the stereochemistry at all stereocenters is that of the naturally-occurring amino acid and naturally-occurring prostaglandin.

In some embodiments, the amino acid is a basic amino acid.

The present specification further discloses novel amino acid salts of prostaglandin free acids suchs as those of Formula II below: 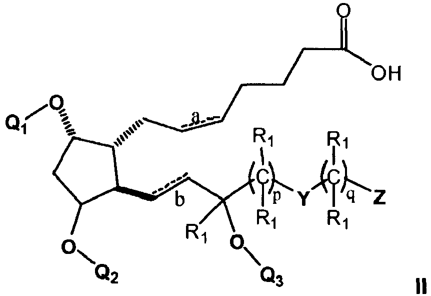 or optical isomers, diastereomers, or enantiomers thereof;
wherein a and b are, independently, single, double or triple bonds;
wherein Q₁, Q₂, and Q₃ are independently selected from hydrogen or an alcohol protecting group;
wherein each R₁ is, independently selected from hydrogen or lower alkyl;
wherein Y is -O-, -S-, -S(O), -SO₂-, -C(R²)₂-, -NR¹-, -CR²=CR²-, or -C=C-;
wherein Z is selected from hydrogen, carbocycle, aryl or heteroaryl;
wherein each R², if present, is independently selected from hydrogen, lower alkyl, alkoxy, or hydroxyl; and
wherein p, and q are independently an integer of from 0 to 4.

Suitably, no carbon atom in **Formula II** has two or more heteroatoms attached to it unless the two or more heteroatoms are member atoms in a heteroaromatic ring system.

In **Formula II** above, the relative stereochemistry at C₈, C₉, and C₁₂ is as specified above. That is, the bond between C₇ and C₈ is in the α orientation, the alcohol (protected or unprotected) at C₉ is in the α orientation, and the bond between C₁₂ and C₁₃ is in the β orientation. The specification also includes optical isomers, diastereomers and enantiomers of the above structure. At all stereocenters where stereochemistry is not defined (e.g. C₁₁ and C₁₅), both epimers are envisioned. In some embodiments stereochemistry at all such stereocenters mimic that of naturally occurring PGF_{2α}.

In some embodiments, Q₁ is either H or an alcohol protecting group and Q₂ and Q₃ are alcohol protecting groups. In other embodiments, Q₁, Q₂, and Q₃ are all alcohol protecting groups and may be different alcohol protecting groups and may be the same alcohol protecting group.

The prostaglandin free acids recited in claim 1 are latanoprost free acid and travoprost free acid. 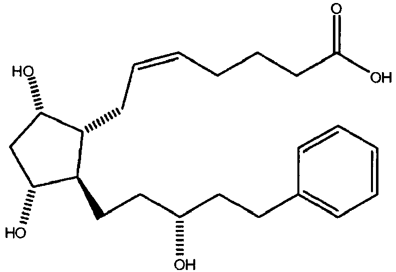 latanoprost free acid 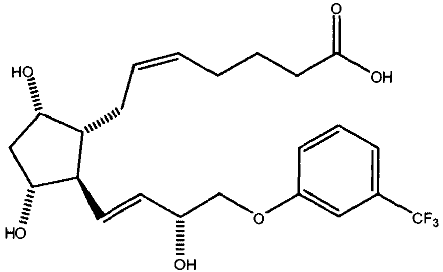 travoprost free acid

The amino acid prostaglandin salts of the present invention are readily formulatable and recrystallizable. They generally are dry free-flowing powders with relatively low tackiness and thus are useful in manufacture. Suitably, the salts have a melting point of at least about 35°C or at least about 50°C.

### Process for Making Amino Acid Prostaglandin Salts

It has surprisingly been discovered that the disadvantages of the difficult procedures used to solidify prostaglandin free acids can be overcome by making a basic amino acid salt of the free acid which can be easily crystallized and/or recrystallized as needed.

The general process for making the amino acid prostaglandin salts according to the present invention is depicted below in **Scheme I**:

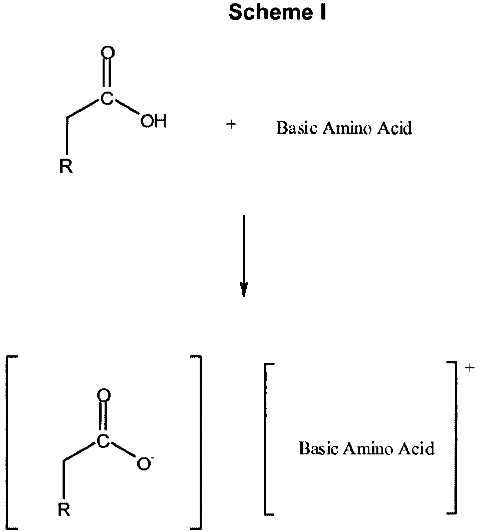

A prostaglandin free acid is reacted with an amino acid in an appropriate solvent to generate the amino acid prostaglandin salt. Solvents can vary but methanol, ethanol, water, THF are generally acceptable solvents.

The temperature of the reaction is suitably kept between -78°C and the ambient room temperature (about 20-25°C). Alternatively, the temperature is from about 0°C to about 20°C. The reaction is stirred for about 5 minutes to about 4 hours under these conditions until a precipitate forms, at which point the amino acid prostaglandin salt is removed by filtration.

The salt may be recrystallized by well-known methods in the art. One suitable method is to dissolve the salt in an alcohol, such as methanol, ethanol or isopropanol, and then add diethyl ether or ethyl acetate until the cloud point, then cool to create a precipitate. The nascent precipitate is then warmed to resolubilize the material and then slowly cooled to create crystals.

Prostaglandin free acids can be made from known starting materials and methods known to one of ordinary skill in the art. For example, many are also available from Cayman Chemical Company, Ann Arbor, MI. In addition, the following reference describes the synthesis of prostaglandins: Corey, E. J.; Weinshenker, N. M.; Schaaf, T. K.; Huber, W J. Am. Chem. Soc., 1969, 91, 5675 and Corey, E. J.; Schaaf, T. K.; Huber, W; Koelliker, U.; Weinshenker, N. M.; J. Am. Chem. Soc., 1970, 92, 397.

### Methods of Treatments Using Amino Acid Prostaglandin Salts

The amino acid prostaglandin salts may be used to treat various conditions, including eye disorders, such as glaucoma, osteoporosis, improving nasal patency or treating neurogenic bladder. The amino acid prostaglandin salts of the present invention are also useful in a method of reducing or decreasing intraocular pressure.

In one embodiment, a cell is contacted with an amount of an amino acid prostaglandin salt effective to reduce intraocular pressure. The term "contacting a cell" is used to mean contacting a cell *in vitro* or *in vivo* (i.e., in a subject, such as a mammal, including humans, rabbits, cats and dogs). In some embodiments, the cell may be contacted as a result of administration of an amino acid prostaglandin salt to a subject. The term "administration" contemplates any route known to one of ordinary skill in the art, including, oral, topical, parenteral, injection, inhalation, implants, buccal and rectal.

An effective amount of an amino acid prostaglandin salt according to the present invention will vary with the particular condition being treated, the age and physical condition of the patient being treated, the severity of the condition, the duration of treatment, the nature of concurrent therapy, the route of administration, the particular pharmaceutically-acceptable carrier utilized, and like factors within the knowledge and expertise of the attending physician. For example, an effective amount of the amino acid prostaglandin salts of the present invention for systemic administration is from about 0.01 to about 1000 µg/kg body weight, preferably from about 0.1 to about 100 µg/kg per body weight, most preferably form about 1 to about 50 µg/kg body weight per day. Transdermal dosages would be designed to attain similar serum or plasma levels, based upon techniques known to those skilled in the art of pharmacokinetics and transdermal formulations. Plasma levels for systemic administration are expected to be in the range of 0.01 to 100 ng/mL, more preferably from 0.05 to 50 ng/mL and most preferably from 0.1 to 10 ng/mL. While these dosages are based upon a daily administration rate, the amino acid prostaglandin salts of the present invention may also be administered at other intervals, such as twice per day, twice weekly, once weekly, or once a month. One of ordinary skill in the art would be able to calculate suitable effective amounts for other intervals of administration.

### Compositions Comprising Amino Acid Prostaglandin Salts

In one embodiment, the amino acid prostaglandin salts are administered in a pharmaceutically acceptable composition, such as in or with a pharmaceutically acceptable carrier.

Compositions may include one or more of the isoforms of the amino acid prostaglandin salts of the present invention. When racemates exists, each enantiomer or diastereomer may be separately used, or they may be combined in any proportion. Where tautomers exist all possible tautomers are specifically contemplated.

Pharmaceutical compositions for use in accordance with the present invention may be formulated in a conventional manner using one or more physiologically acceptable carriers or excipients. Thus, the amino acid prostaglandin salts may be formulated for administration by, for example, solid dosing, eyedrop, in a topical oil-based formulation, injection, inhalation (either through the mouth or the nose), implants, or oral, buccal, parenteral or rectal administration. Techniques and formulations may generally be found in "Remington's Pharmaceutical Sciences", (Meade Publishing Co., Easton, Pa.).

The route by which the amino acid prostaglandin salts of the present invention (component A) will be administered and the form of the composition will dictate the type of carrier (component B) to be used. The composition may be in a variety of forms, suitable, for example, for systemic administration (e.g., oral, rectal, nasal, sublingual, buccal, implants, or parenteral) or topical administration (e.g., local application on the skin, ocular, liposome or nanosphere delivery systems, or iontophoresis).

Carriers for systemic administration typically comprise at least one of a) diluents, b) lubricants, c) binders, d) disintegrants, e) colorants, f) flavors, g) sweeteners, h) antioxidants, j) preservatives, k) glidants, m) solvents, n) suspending agents, o) wetting agents, p) surfactants, combinations thereof, and others. All carriers are optional in the systemic compositions.
Ingredient a) is a diluent. Suitable diluents for solid dosage forms include sugars such as glucose, lactose, dextrose, and sucrose; diols such as propylene glycol; calcium carbonate; sodium carbonate; sugar alcohols, such as glycerin; mannitol; and sorbitol. The amount of ingredient a) in the systemic or topical composition is typically about 50 to about 90%.
Ingredient b) is a lubricant. Suitable lubricants for solid dosage forms are exemplified by solid lubricants including silica, talc, stearic acid and its magnesium salts and calcium salts, calcium sulfate; and liquid lubricants such as polyethylene glycol and vegetable oils such as peanut oil, cottonseed oil, sesame oil, olive oil, corn oil and oil of theobroma. The amount of ingredient b) in the systemic or topical composition is typically about 5 to about 10%.
Ingredient c) is a binder. Suitable binders for solid dosage forms include polyvinyl pyrrolidone; magnesium aluminum silicate; starches such as corn starch and potato starch; gelatin; tragacanth; and cellulose and its derivatives, such as sodium carboxymethylcellulose, ethyl cellulose, methylcellulose, microcrystalline cellulose, and sodium carboxymethylcellulose. The amount of ingredient c) in the systemic composition is typically about 5 to about 50%, and in ocular solid dosing forms up to 99%.
Ingredient d) is a disintegrant. Suitable disintegrants for solid dosage forms include agar, alginic acid and the sodium salt thereof, effervescent mixtures, croscarmelose, crospovidone, sodium carboxymethyl starch, sodium starch glycolate, clays, and ion exchange resins. The amount of ingredient d) in the systemic or topical composition is typically about 0.1 to about 10%.
Ingredient e) for solid dosage forms is a colorant such as an FD&C dye. When used, the amount of ingredient e) in the systemic or topical composition is typically about 0.005 to about 0.1%.
Ingredient f) for solid dosage forms is a flavor such as menthol, peppermint, and fruit flavors. The amount of ingredient f), when used, in the systemic or topical composition is typically about 0.1 to about 1.0%.
Ingredient g) for solid dosage forms is a sweetener such as aspartame and saccharin. The amount of ingredient g) in the systemic or topical composition is typically about 0.001 to about 1%.
Ingredient h) is an antioxidant such as butylated hydroxyanisole ("BHA"), butylated hydroxytoluene ("BHT"), and vitamin E. The amount of ingredient h) in the systemic or topical composition is typically about 0.1 to about 5%.
Ingredient j) is a preservative such as benzalkonium chloride, methyl paraben and sodium benzoate. The amount of ingredient j) in the systemic or topical composition is typically about 0.001 to about 5%, suitably about 0.01 to about 5%.
Ingredient k) for solid dosage forms is a glidant such as silicon dioxide. The amount of ingredient k) in the systemic or topical composition is typically about 1 to about 5%.
Ingredient m) is a solvent, such as water, isotonic saline, ethyl oleate, glycerine, hydroxylated castor oils, alcohols such as ethanol, and phosphate buffer solutions. The amount of ingredient m) in the systemic or topical composition is typically from about 0 to about 100%.
Ingredient n) is a suspending agent. Suitable suspending agents include Avicel® RC-591 (from FMC Corporation of Philadelphia, PA) and sodium alginate. The amount of ingredient n) in the systemic or topical composition is typically about 1 to about 8%.
Ingredient o) is a surfactant such as lecithin, Polysorbate 80, and sodium lauryl sulfate, and the TWEENS® from Atlas Powder Company of Wilmington, Delaware. Suitable surfactants include those disclosed in the C.T.F.A. Cosmetic Ingredient Handbook, 1992, pp.587-592; Remington's Pharmaceutical Sciences, 15th Ed. 1975, pp. 335-337; and McCutcheon's Volume 1, Emulsifiers & Detergents, 1994, North American Edition, pp. 236-239. The amount of ingredient o) in the systemic or topical composition is typically about 0.1% to about 5%.

Although the amounts of components A and B in the systemic compositions will vary depending on the type of systemic composition prepared, the specific derivative selected for component A and the ingredients of component B, in general, system compositions comprise 0.01% to 50% of component A and 50 to 99.99% of component B.

Compositions for parenteral administration typically comprise A) 0.1 to 10% of the amino acid prostaglandin salts of the present invention and B) 90 to 99.9% of a carrier comprising a) a diluent and m) a solvent. In one embodiment, component a) comprises propylene glycol and m) comprises ethanol or ethyl oleate.

Compositions for oral administration can have various dosage forms. For example, solid forms include tablets, capsules, granules, and bulk powders. These oral dosage forms comprise a safe and effective amount, usually at least about 5%, and more particularly from about 25% to about 50% of component A). The oral dosage compositions further comprise about 50 to about 95% of component B), and more particularly, from about 50 to about 75%.

Tablets can be compressed, tablet triturates, enteric-coated, sugar-coated, film-coated, or multiple-compressed. Tablets typically comprise component A, and component B a carrier comprising ingredients selected from the group consisting of a) diluents, b) lubricants, c) binders, d) disintegrants, e) colorants, f) flavors, g) sweeteners, k) glidants, and combinations thereof. Specific diluents include calcium carbonate, sodium carbonate, mannitol, lactose and cellulose. Specific binders include starch, gelatin, and sucrose. Specific disintegrants include alginic acid and croscarmelose. Specific lubricants include magnesium stearate, stearic acid, and talc. Specific colorants are the FD&C dyes, which can be added for appearance. Chewable tablets preferably contain g) sweeteners such as aspartame and saccharin, or f) flavors such as menthol, peppermint, fruit flavors, or a combination thereof.

Capsules (including implants, time release and sustained release formulations) typically comprise component A, and a carrier comprising one or more a) diluents disclosed above in a capsule comprising gelatin. Granules typically comprise component A, and preferably further comprise k) glidants such as silicon dioxide to improve flow characteristics. Implants can be of the biodegradable or the non-biodegradable type. Implants may be prepared using any known biocompatible formulation.

The selection of ingredients in the carrier for oral compositions depends on secondary considerations like taste, cost, and shelf stability, which are not critical for the purposes of this invention. One skilled in the art would know how to select appropriate ingredients without undue experimentation.

The solid compositions may also be coated by conventional methods, typically with pH or time-dependent coatings, such that component A is released in the gastrointestinal tract in the vicinity of the desired application, or at various points and times to extend the desired action. The coatings typically comprise one or more components selected from the group consisting of cellulose acetate phthalate, polyvinyl acetate phthalate, hydroxypropyl methyl cellulose phthalate, ethyl cellulose, EUDRAGIT® coatings (available from Rohm & Haas G.M.B.H. of Darmstadt, Germany), waxes and shellac.

Compositions for oral administration can also have liquid forms. For example, suitable liquid forms include aqueous solutions, emulsions, suspensions, solutions reconstituted from non-effervescent granules, suspensions reconstituted from non-effervescent granules, effervescent preparations reconstituted from effervescent granules, elixirs, tinctures, syrups, and the like. Liquid orally administered compositions typically comprise component A and component B, namely, a carrier comprising ingredients selected from the group consisting of a) diluents, e) colorants, f) flavors, g) sweeteners, j) preservatives, m) solvents, n) suspending agents, and o) surfactants. Peroral liquid compositions preferably comprise one or more ingredients selected from the group consisting of e) colorants, f) flavors, and g) sweeteners.

Other compositions useful for attaining systemic delivery of the subject amino acid prostaglandin salts include sublingual, buccal and nasal dosage forms. Such compositions typically comprise one or more of soluble filler substances such as a) diluents including sucrose, sorbitol and mannitol; and c) binders such as acacia, microcrystalline cellulose, carboxymethyl cellulose, and hydroxypropyl methylcellulose. Such compositions may further comprise b) lubricants, e) colorants, f) flavors, g) sweeteners, h) antioxidants, and k) glidants.

In one embodiment of the invention, the amino acid prostaglandin salts of the present invention are topically administered. Topical compositions that can be applied locally to the eye may be in any form known in the art, non-limiting Examples of which include solids, gelable drops, sprays, ointments, or a sustained or non-sustained release unit placed in the conjunctival cul-du-sac of the eye or another appropriate location.

Topical compositions that can be applied locally to the skin may be in any form including solids, solutions, oils, creams, ointments, gels, lotions, shampoos, leave-on and rinse-out hair conditioners, milks, cleansers, moisturizers, sprays, skin patches, and the like. Topical compositions comprise: component A, the amino acid prostaglandin salts described above, and component B, a carrier. The carrier of the topical composition preferably aids penetration of the amino acid prostaglandin salts into the eye. Component B may further comprise one or more optional components.

The exact amounts of each component in the topical composition depend on various factors. The amount of component A added to the topical composition is dependent on the IC50 of component A, typically expressed in nanomolar (nM) units. For example, if the IC50 of the medicament is 1 nM, the amount of component A will be from about 0.001 to about 0.3%. If the IC50 of the medicament is 10 nM, the amount of component A) will be from about 0.01 to about 1%. If the IC50 of the medicament is 100 nM, the amount of component A will be from about 0.1 to about 10%. If the IC50 of the medicament is 1000 nM, the amount of component A will be 1 to 100%, preferably 5% to 50%. If the amount of component A is outside the ranges specified above (i.e., lower), efficacy of the treatment may be reduced. One skilled in the art understands how to calculate and understand an IC50. The remainder of the composition, up to 100%, is component B.

The amount of the carrier employed in conjunction with component A is sufficient to provide a practical quantity of composition for administration per unit dose of the medicament. Techniques and compositions for making dosage forms useful in the methods of this invention are described in the following references: Modern Pharmaceutics, Chapters 9 and 10, Banker & Rhodes, eds. (1979); Lieberman et al., Pharmaceutical Dosage Forms: Tablets (1981); and Ansel, Introduction to Pharmaceutical Dosage Forms, 2nd Ed., (1976).

Component B may comprise a single ingredient or a combination of two or more ingredients. In the topical compositions, component B comprises a topical carrier. Suitable topical carriers comprise one or more ingredients selected from the group consisting of phosphate buffered saline, isotonic water, deionized water, monofunctional alcohols, symmetrical alcohols, aloe vera gel, allantoin, glycerin, vitamin A and E oils, mineral oil, propylene glycol, PPG-2 myristyl propionate, dimethyl isosorbide, castor oil, combinations thereof, and the like. More particularly, carriers for skin applications include propylene glycol, dimethyl isosorbide, and water, and even more particularly, phosphate buffered saline, isotonic water, deionized water, monofunctional alcohols and symmetrical alcohols.

The carrier of the topical composition may further comprise one or more ingredients selected from the group consisting of q) emollients, r) propellants, s) solvents, t) humectants, u) thickeners, v) powders, w) fragrances, x) pigments, and y) preservatives.
Ingredient q) is an emollient. The amount of ingredient q) in a skin-based topical composition is typically about 5 to about 95%. Suitable emollients include stearyl alcohol, glyceryl monoricinoleate, glyceryl monostearate, propane-1,2-diol, butane-1,3-diol, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, cetyl palmitate, di-n-butyl sebacate, isopropyl myristate, isopropyl palmitate, isopropyl stearate, butyl stearate, polyethylene glycol, triethylene glycol, lanolin, sesame oil, coconut oil, arachis oil, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate, and combinations thereof. Specific emollients for skin include stearyl alcohol and polydimethylsiloxane.
Ingredient r) is a propellant. The amount of ingredient r) in the topical composition is typically about 0 to about 95%. Suitable propellants include propane, butane, isobutane, dimethyl ether, carbon dioxide, nitrous oxide, and combinations thereof.
Ingredient s) is a solvent. The amount of ingredient s) in the topical composition is typically about 0 to about 95%. Suitable solvents include water, ethyl alcohol, methylene chloride, isopropanol, castor oil, ethylene glycol monoethyl ether, diethylene glycol monobutyl ether, diethylene glycol monoethyl ether, dimethylsulfoxide, dimethyl formamide, tetrahydrofuran, and combinations thereof. Specific solvents include water, ethyl alcohol and propylene glycol.
Ingredient t) is a humectant. The amount of ingredient t) in the topical composition is typically 0 to 95%. Suitable humectants include glycerin, sorbitol, sodium 2-pyrrolidone-5-carboxylate, soluble collagen, dibutyl phthalate, gelatin, and combinations thereof. Specific humectants include glycerin.
Ingredient u) is a thickener. The amount of ingredient u) in the topical composition is typically about 0 to about 95%.
Ingredient v) is a powder. The amount of ingredient v) in the topical composition is typically 0 to 95%. Suitable powders include beta-cyclodextrins, hydroxypropyl cyclodextrins, chalk, talc, fullers earth, kaolin, starch, gums, colloidal silicon dioxide, sodium polyacrylate, tetra alkyl ammonium smectites, trialkyl aryl ammonium smectites, chemically-modified magnesium aluminum silicate, organically-modified Montmorillonite clay, hydrated aluminum silicate, fumed silica, carboxyvinyl polymer, sodium carboxymethyl cellulose, ethylene glycol monostearate, and combinations thereof. For ocular applications, specific powders include beta-cyclodextrin, hydroxypropyl cyclodextrin, and sodium polyacrylate. For gel dosing ocular formulations, sodium polyacrylate may be used.
Ingredient w) is a fragrance. The amount of ingredient w) in the topical composition is typically about 0 to about 0.5%, particularly, about 0.001 to about 0.1%. For ocular applications a fragrance is not typically used.
Ingredient x) is a pigment. Suitable pigments for skin applications include inorganic pigments, organic lake pigments, pearlescent pigments, and mixtures thereof. Inorganic pigments useful in this invention include those selected from the group consisting of rutile or anatase titanium dioxide, coded in the Color Index under the reference CI 77,891; black, yellow, red and brown iron oxides, coded under references CI 77,499, 77,492 and, 77,491; manganese violet (CI 77,742); ultramarine blue (CI 77,007); chromium oxide (CI 77,288); chromium hydrate (CI 77,289); and ferric blue (CI 77,510) and mixtures thereof.

The organic pigments and lakes useful in this invention include those selected from the group consisting of D&C Red No. 19 (CI 45,170), D&C Red No. 9 (CI 15,585), D&C Red No. 21 (CI 45,380), D&C Orange No. 4 (CI 15,510), D&C Orange No. 5 (CI 45,370), D&C Red No. 27 (CI 45,410), D&C Red No. 13 (CI 15,630), D&C Red No. 7 (CI 15,850), D&C Red No. 6 (CI 15,850), D&C Yellow No. 5 (CI 19,140), D&C Red No. 36 (CI 12,085), D&C Orange No. 10 (CI 45,425), D&C Yellow No. 6 (CI 15,985), D&C Red No. 30 (CI 73,360), D&C Red No. 3 (CI 45,430), the dye or lakes based on Cochineal Carmine (CI 75,570) and mixtures thereof.

The pearlescent pigments useful in this invention include those selected from the group consisting of the white pearlescent pigments such as mica coated with titanium oxide, bismuth oxychloride, colored pearlescent pigments such as titanium mica with iron oxides, titanium mica with ferric blue, chromium oxide, titanium mica with an organic pigment of the above-mentioned type as well as those based on bismuth oxychloride and mixtures thereof. The amount of pigment in the topical composition is typically about 0 to about 10%. For ocular applications a pigment is generally not used.

In a particularly preferred embodiment of the invention, topical pharmaceutical compositions for ocular administration are prepared typically comprising component A and B (a carrier), such as purified water, and one or more ingredients selected from the group consisting of y) sugars or sugar alcohols such as dextrans, particularly mannitol and dextran 70, z) cellulose or a derivative thereof, aa) a salt, bb) disodium EDTA (Edetate disodium), and cc) a pH adjusting additive.

Examples of z) cellulose derivatives suitable for use in the topical pharmaceutical composition for ocular administration include sodium carboxymethylcellulose, ethylcellulose, methylcellulose, and hydroxypropyl-methylcellulose, particularly, hydroxypropyl-methylcellulose.

Examples of aa) salts suitable for use in the topical pharmaceutical composition for ocular administration include mono-, di- and trisodium phosphate, sodium chloride, potassium chloride, and combinations thereof.

Examples of cc) pH adjusting additives include HCI or NaOH in amounts sufficient to adjust the pH of the topical pharmaceutical composition for ocular administration to 5.0-7.5.

Component A may be included in kits comprising component A, a systemic or topical composition described above, or both; and information, instructions, or both that use of the kit will provide treatment for cosmetic and medical conditions in mammals (particularly humans). The information and instructions may be in the form of words, pictures, or both, and the like. In addition or in the alternative, the kit may comprise the medicament, a composition, or both; and information, instructions, or both, regarding methods of application of medicament, or of composition, preferably with the benefit of treating or preventing cosmetic and medical conditions in mammals (e.g., humans).

### EXAMPLES

The following examples further illustrate the processes of the present invention:

### Example 1

### Preparation of latanoprost arginine salt (E1):

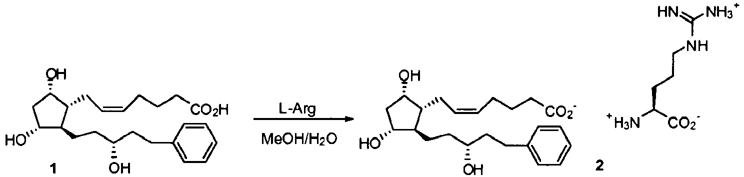

To Latanoprost free acid (CAS # 41639-83-2) (**1**, 21.2 mg, 0.054 mmol) in MeOH/H₂O (4:1, 0.65 mL) was added L-Arginine (>98%, Sigma-Aldrich, St. Louis, MO). (72 mM solution in MeOH/H₂O [4:1]) (754 µL, 0.054 mmol) and the solution was stirred for 30 minutes. The solvents were evaporated then azeotroped with Et₂O and hexanes to give white crystalline Latanoprost-L-Arginine salt **2** (quantitative).

*Recrystallization:* To 26.5 mg of **1** in a **4** mL vial was added approx 0.5 mL of MeOH to dissolve, followed by 1.2-1.5 mL of Et₂O which precipitated out the product. The vial was put on ice for 5 min. The liquid was decanted and the solid dried to give pure **2**, 22.9 mg.

### Example 2 (Reference Example)

Using substantially the same procedure, but substituting PGE₂ free acid (CAS #: 363-24-6) for latanoprost free acid the arginine salt of PGE₂ can be made.

### Examples 3-71 (Examples 3-12 and 15-71 are Reference Examples)

Arginine salts of the following commercially-available prostaglandins can be made by following the above procedure and substituting the appropriate prostaglandin free acid.

| **Example** | **Free acid name** | **CAS #** |
|---|---|---|
| 3 | 8-iso Prostaglandin F2α | 27415-26-5 |
| 4 | Prostaglandin D2 | 41598-07-6 |
| 5 | Prostaglandin E1 | 745-65-3 |
| 6 | Prostaglandin F2α | 551-11-1 |
| 7 | Prostaglandin D3 | 71902-47-1 |
| 8 | 16,16-dimethyl Prostaglandin E2 | 39746-25-3 |
| 9 | Butaprost (free acid) | 433219-55-7 |
| 10 | Tafluprost (free acid) | 209860-88-8 |
| 11 | Bimatoprost (free acid) | |
| 12 | Prostaglandin A1 | 14152-28-4 |
| 13 | Travoprost (free acid) | |
| 14 | Latanoprost (free acid)-d4 | |
| 15 | 17-phenyl trinor Prostaglandin E2 | 38315-43-4 |
| 16 | 15(S)-Latanoprost (free acid) | |
| 17 | AL 8810 | 246246-19-5 |
| 18 | Bimatoprost (free acid) | 38344-08-0 |
| 19 | (+)-15-epi Cloprostenol | 54276-22-1 |
| 20 | 15-keto Latanoprost (free acid) | |
| 21 | Prostaglandin E3 | 802-31-3 |
| 22 | Limaprost | 74397-12-9 |
| 23 | (+)-Fluprostenol (also know as travoprost free acid) | 54276-17-4 |
| 24 | BW 245C | 72814-32-5 |
| 25 | Δ12-Prostaglandin J2 | 87893-54-7 |
| 26 | 11β-Prostaglandin F2α | 38432-87-0 |
| 27 | 5-trans Prostaglandin E2 | 36150-00-2 |
| 28 | 19(R)-hydroxy Prostaglandin E2 | 64625-54-3 |
| 29 | Fluprostenol (racemic) | 40666-16-8 |
| 30 | Prostaglandin F2β | 4510-16-1 |
| 31 | 16-phenoxy tetranor Prostaglandin E2 | |
| 32 | (+)-Cloprostenol | 54276-21-0 |
| 33 | 20-hydroxy Prostaglandin E2 | 57930-95-7 |
| 34 | 17,20-dimethyl Prostaglandin F1α | |
| 35 | (+)-Cloprostenol | |
| 36 | 9-keto Fluprostenol | 156406-33-6 |
| 37 | Prostaglandin F1α | 745-62-0 |
| 38 | 15(S)-Fluprostenol | |
| 39 | 5-trans Prostaglandin F2β | 36150-02-4 |
| 40 | 15-keto-17-phenyl trinor Prostaglandin F2α | |
| 41 | 15-cyclohexyl pentanor Prostaglandin F2α | 58611-97-5 |
| 42 | Cloprostenol (racemic) | |
| 43 | Prostaglandin F1 β | 10164-73-5 |
| 44 | 3-methoxy Limaprost | |
| 45 | 13,14-dihydro Prostaglandin F2α | 27376-74-5 |
| 46 | 15(R)-15-methyl Prostaglandin E2 | 55028-70-1 |
| 47 | 19(R)-hydroxy Prostaglandin E1 | 64625-55-4 |
| 48 | 9-deoxy-9-methylene Prostaglandin E2 | 61263-32-9 |
| 49 | 16-phenyl tetranor Prostaglandin E1 | |
| 50 | 11-deoxy Prostaglandin F1α | 37785-98-1 |
| 51 | 19(R)-hydroxy Prostaglandin F1α | 81371-59-7 |
| 52 | 19(R)-hydroxy Prostaglandin F2α | 64625-53-2 |
| 53 | 1a, 1b-dihomo Prostaglandin F2α | 57944-39-5 |
| 54 | 15(R), 19(R)-hydroxy Prostaglandin F2α | |
| 55 | 16-phenoxy tetranor Prostaglandin F2α | 51705-19-2 |
| 56 | 11-deoxy Prostaglandin F1β | 37785-99-2 |
| 57 | 11-deoxy Prostaglandin F2β | 37786-07-5 |
| 58 | 11β-Prostaglandin F1β | 37785-86-7 |
| 59 | 15-keto Fluprostenol | |
| 60 | 15(S)-15-methyl Prostaglandin F2α | 35700-23-3 |
| 61 | 16,16-dimethyl Prostaglandin E1 | 41692-15-3 |
| 62 | 16,16-dimethyl Prostaglandin F2α | 39746-23-1 |
| 63 | 17-trifluoromethylphenyl-13,14-dihydro trinor Prostaglan | |
| 64 | 17-trifluoromethylphenyl trinor Prostaglandin F2α | 221246-34-0 |
| 65 | Bimatoprost (free acid)-d4 | |
| 66 | (+)-Fluprostenol-d4 | |
| 67 | Prostaglandin B2 | 13367-85-6 |
| 68 | Prostaglandin B3 | 36614-32-1 |
| 69 | 17-phenyl trinor Prostaglandin A2 | |
| 70 | (S)-AL 8810 | |
| 71 | Lubiprostone | 136790-76-6 |

### Example 72

To Latanoprost (free acid) (**1**, 21.2 mg, 0.054 mmol) in MeOH/H₂O (4:1, 0.65 mL) was added L-Arginine (72 mM solution in MeOH/H₂O [4:1]) (754 µL, 0.054 mmol) ((>98%) from Sigma-Aldrich Cat. A5006) and the solution was stirred for 30 minutes. The solvents were evaporated then azeotroped with Et₂O and hexanes to give white crystalline Latanoprost-L-Arginine salt **2** (quantitative).

*Recrystallization:* To 26.5 mg of **1** in a 4 mL vial was added approx 0.5 mL of MeOH to dissolve, followed by 1.2-1.5 mL of Et₂O which precipitated out the product. The vial was put on ice for 5 min. The liquid was decanted and the solid dried to give pure **2**, 22.9 mg.

### Example 73

To travoprost free acid ((+)-fluprostenol free acid) **3** in MeOH/H₂O was added L-Arginine (>98%) (Sigma-Aldrich Cat. A5006) and the solution was stirred for 30 minutes. The solvents were evaporated then azeotroped with Et₂O and hexanes to give white crystalline travoprost-L-Arginine salt **4** (quantitative).

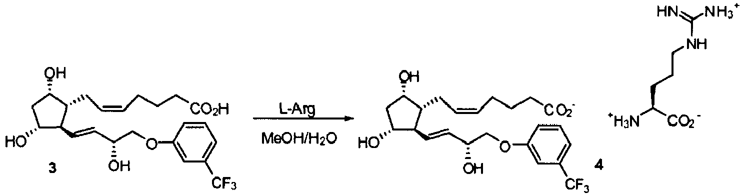

*Recrystallization:* To **4** is added enough MeOH to dissolve, and is followed by Et₂O to the cloud point, then warmed to redissolved and slowly cooled overnight. The liquid is decanted and the solid is dried to give pure **4**.

### Example 74 (Reference Example)

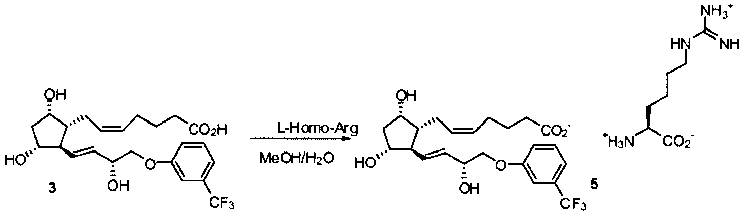

To travoprost free acid ((+)-fluprostenol free acid) **3** in MeOH/H₂O is added L-Homoarginine and the solution is stirred for 30 minutes. The solvents are evaporated then azeotroped with Et₂O and hexanes to give white crystalline travoprost-L-Homoarginine salt **5** (quantitative).

*Recrystallization:* To **5** was added enough EtOH to dissolve, followed by Et₂O which precipitated the product. The vial was put on ice for 5 min. The liquid was decanted and the solid dried to give the salt **5**.

### Example 75

### Formulation of Liquid Compositions Comprising Present Compounds

A composition in liquid form is prepared by conventional methods, formulated as follows:

### Example 75: Preparation Table

| Ingredient | Quantity |
|---|---|
| *PGF analog arginine salt* | 0-5 mg |
| PGE analog arginine salt | 0-5 mg |
| Propylene glycol | 5 mL |
| Ethyl alcohol | 5 mL |

1.0 mL of the above composition, when administered once a day, substantially increases the beauty and health of the mammalian skin onto which it is applied.

### Example 76

### Preparation of Skin Care Topical Product Comprising Present Compounds

A skin care, topical product is prepared by formulating a cream composition as illusterated below.

### Example 76: Preparation Table

| Ingredient | Quantity |
|---|---|
| *PGF analog arginine salt* | 50 mg |
| Isopropyl isosterate | 150 g |
| Polyacrylate 13/polyisobutene/Polysorbate 20 | 35 g |
| Methyl paraben/propyl paraben | 1 g |
| Distilled water | 400 g |

### Example 77

### Preparation of Skin Care Wipe Product Comprising Present Compounds

A skin care wipe product is prepared by impregnating such a wipe with the liquid composition of Example 75. Such a wipe may be impregnated by techniques known and readily available to those skilled in the art. Indeed, a preferred example of a wipe product is the Oil of Olay Facial Wipes, owned and distributed by The Procter and Gamble Company of Cincinnati, Ohio.

### Example 78

Shampoos are made, comprising:

| | Ex. 78-1 | Ex. 78-2 | Ex. 78-3 | Ex. 78-4 |
|---|---|---|---|---|
| Component | | | | |
| Ammonium Lauryl Sulfate | 11.5 % | 11.5 % | 9.5 % | 7.5 % |
| Ammonium Laureth Sulfate | 4 % | 3 % | 2 % | 2 % |
| Cocamide MEA | 2 % | 2 % | 2 % | 2 % |
| Ethylene Glycol Distearate | 2 % | 2 % | 2 % | 2 % |
| Cetyl Alcohol | 2 % | 2 % | 2 % | 2 % |
| Stearyl Alcohol | 1.2 % | 1.2 % | 1.2 % | 1.2 % |
| Glycerin | 1 % | 1 % | 1 % | 1 % |
| Polyquaternium 10 | 0.5 % | 0.25 % | - | - |
| Polyquaternium 24 | - | - | 0.5 % | 0.25 % |
| Sodium Chloride | 0.1 % | 0.1 % | 0.1 % | 0.1 % |
| Sucrose Polyesters of Cottonate Fatty Acid | 3 % | 3 % | - | - |
| Sucrose Polyesters of Behenate Fatty Acid | 2% | 3 % | - | - |
| Polydimethyl Siloxane | - | - | 3 % | 2% |
| Cocaminopropyl Betaine | - | 1 % | 3% | 3% |
| Lauryl Dimethyl Amine Oxide | 1.5 % | 1.5 % | 1.5 % | 1.5 % |
| Decyl Polyglucose | - | - | 1 % | 1 % |
| DMDM Hydantoin | 0.15 % | 0.15 % | 0.15 % | 0.15 % |
| PGF analog arginine salt | - | 0.2 % | 0.2 % | - |
| PGE analog arginine salt | 0.1 % | - | - | 0.1 % |
| Minoxidil | | | 3 % | 2 % |
| Phenoxyethanol | 0.5 % | 0.5 % | 0.5 % | 0.5 % |
| Fragrance | 0.5 % | 0.5 % | 0.5 % | 0.5 % |
| Water | q.s. | q.s. | q.s. | q.s. |

A human subject suffering from male pattern baldness is treated by a method of this invention. Specifically, for 12 weeks, a shampoo described above is used daily by the subject. Approximately one liquid ounce of the formula is applied to the scalp and hair of the subject, left in place for not more than 5 minutes, then rinsed with water.

### Example 79

Topical pharmaceutical compositions for lowering intraocular pressure are prepared by conventional methods and formulated as follows:

| Ingredient | Amount (wt %) |
|---|---|
| *PGF analog arginine salt* | 0.50 |
| Dextran 70 | 0.1 |
| Hydroxypropyl methylcellulose | 0.3 |
| Sodium Chloride | 0.77 |
| Potassium chloride | 0.12 |
| Disodium EDTA | 0.05 |
| Benzalkonium chloride | 0.01 |
| HCl and/or NaOH | pH 7.0-7.2 |
| Purified water | q.s. to 100% |

A compound according to this invention is used as the PF analog arginine salt. When the composition is topically administered to the eyes as a 40 microliter drop instilled into the affected eye once daily, the above composition decreases intraocular pressure in a patient suffering from glaucoma.

### Example 80

Example 79 is repeated using a PGE analog arginine salt according to this invention. When administered as a drop twice per day, the above composition substantially decreases intraocular pressure.

### Example 81

Topical pharmaceutical compositions for nasal sprays are prepared by conventional methods and formulated as follows:

| Ingredient | Amount (wt %) |
|---|---|
| *PGF analog arginine salt* | 0.1-0.50 |
| Hydroxypropyl methylcellulose | 0.0-0.3 |
| Sorbitol | 0.61 |
| EDTA | 0.05 |
| Benzalkonium chloride | 0.01 |
| HCl and/or NaOH to | pH 7.0-7.2 |
| Purified water | q.s. to 100% |

### Example 82

Topical pharmaceutical compositions for instillation, *e*.*g*., into a bladder are prepared by conventional methods and formulated as follows:

| Ingredient | Amount (wt %) |
|---|---|
| *PGF analog arginine salt* | 0.1-0.50 |
| EDTA | 0.05 |
| Phosphate buffered saline | q.s. to 100% |

### Example 83 (Reference Example)

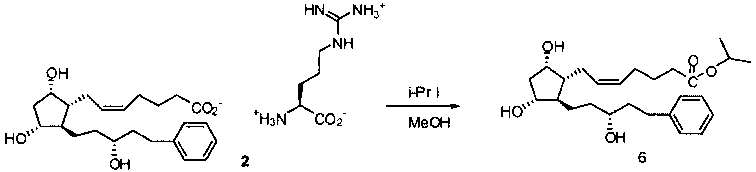

To 1.0 gram of Latanoprost L-arginine salt (2) in MeOH/H₂O (4:1, 20 mL) is added 2.01 equivalents of isopropyl iodide (99%, Cat #148938, Sigma-Aldrich, St. Louis, MO). and the solution is stirred overnight. Saturated aqueous ammonium acetate is added along with ethyl acetate and the layers are separated. The organic layer is separated, and the solvents removed in vaccuo, and the material is chromatographed to give latanoprost as the isopropyl ester (**6**).

### Example 84 (Reference Example)

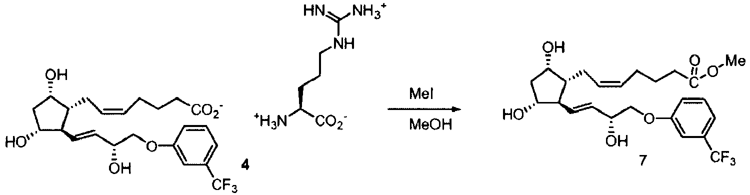

The procedure of example 83 is repeated substituting fluprostenol for latanoprost and methyl iodide for isopropyl iodide. Isolated is fluprostenol methyl ester (**7**)

### Example 85 (Reference Example)

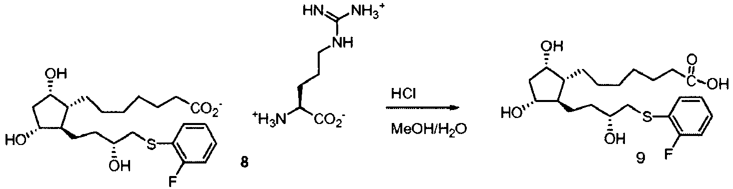

To 1.0 gram of Arginyl 7-((1R,2R,3R,5S)-2-((R)-4-(2-fluorophenylthio)-3-hydroxybutyl)-3,5-dihydroxy cyclopentyl)heptanoate (**8**) in MeOH/H₂O (4:1, 20 mL) is added, dropwise with cooling, at least 5 equivalents of a 3:1 mixture of saturated aqueous ammonium chloride and 1 M hydrochloric acid (Sigma-Aldrich, St. Louis, MO), and the solution is stirred for 15 minutes. Saturated aqueous sodium chloride is added along with ethyl acetate. The organic layer is separated; is washed again with brine; the solvents removed in vaccuo, and the material chromatographed to give the free acid, 7-((1R,2R,3R,5S)-2-((R)-4-(2-fluorophenylthio)-3-hydroxybutyl)-3,5-dihydroxy cyclopentyl)heptanoic acid (**9**).

### Example 86 (Reference Example)

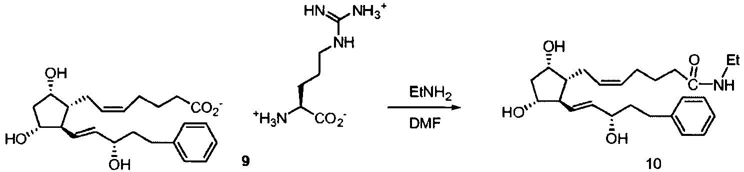

To 1.0 gram of bimataprost arginine salt (9) in DMF (20 mL) is added 2.01 equivalents of 2M ethyl amine solution in THF (Cat #395072, Sigma-Aldrich, St. Louis, MO) and 1.5 eq of EDC. and the solution is stirred overnight. Added is at least 5 equivalents of a 3:1 mixture of saturated aqueous ammonium chloride and 1 M hydrochloric acid (Sigma-Aldrich, St. Louis, MO), and the solution is extracted with ethyl acetate, washed and purified. Isolated is bimataprost *N*-ethyl amide (**10**)

### Example 87 (Reference Example)

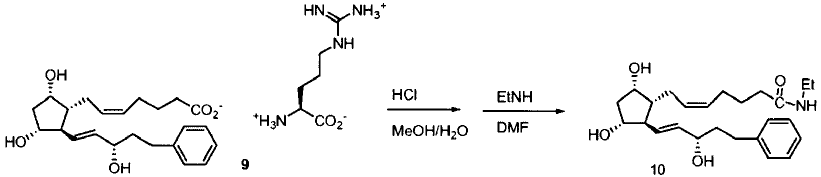

To 1.0 gram of bimataprost arginine salt (**9**) is added at least 5 equivalents of a 3:1 mixture of saturated aqueous ammonium chloride and 1 M hydrochloric acid (Sigma-Aldrich, St. Louis, MO), and the solution is extracted with diethyl ether. The organic layer is then separated, and added are: 20 mL of DMF, 2.01 equivalents of 2M ethyl amine solution in THF (Cat #395072, Sigma-Aldrich, St. Louis, MO), 1.5 eq of EDC and 0.2 eq. of DMAP, and the solution is stirred overnight. Then added is at least 5 equivalents of a 3:1 mixture of saturated aqueous ammonium chloride and 1 M hydrochloric acid (Sigma-Aldrich, St. Louis, MO), and the solution is extracted with ethyl acetate, washed and purified. Isolated is bimataprost *N*-ethyl amide (**10**)

### Example 88

White crystalline Latanoprost-L-Arginine salt **2** was prepared as described in Example 1. Latanoprost-L-Arginine salt **2** was recrystallized by dissolving the salt in 10 volumes of ethanol (under nitrogen) and cooling the solution to approximately -40°C. 30 volumes of methyl *tert*-butyl ether (MTBE) were added slowly to the solution while stirring gently. After the addition was complete, the mixture was stirred for a short period of time at approximately -40°C. Stirring was halted and the salt was allowed to settle. Most of the supernatant was removed by suction using a 100 mL pipet. 20 volumes of (1:3) ethanol:MTBE was added to the remaining mixture in the flask and stirred at approximately -40°C for a few minutes. Stirring was halted and the salt was allowed to settle. Most of the supernatant was removed by suction using a 100 mL pipet. 20 volumes of MTBE was added to the remaining mixture and stirred at approximately -40°C for a few minutes. Stirring was halted and the salt was allowed to settle. Most of the supernatant was removed by suction using a 100 mL pipet. 20 volumes of MTBE was added to the remaining mixture and stirred at approximately -40°C for a few minutes. The mixture was filtered and the solid was washed with 10 volumes of MTBE. The solid was placed a crystallizing dish in the vacuum oven under hi vac for drying.

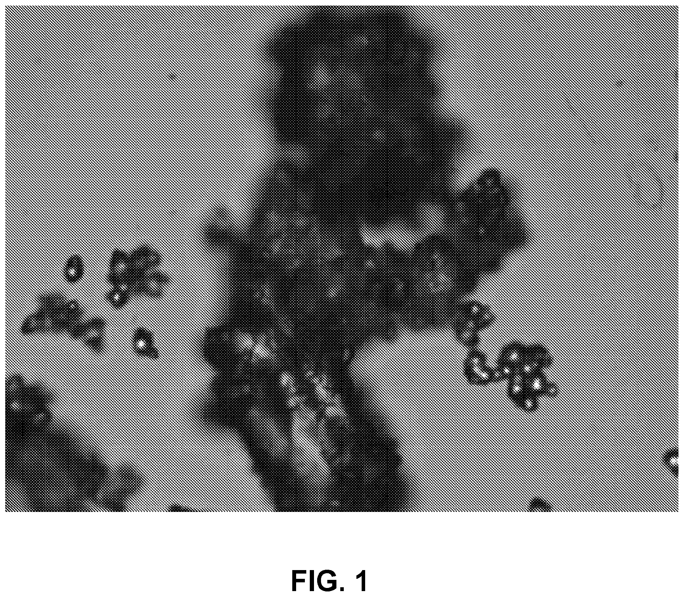

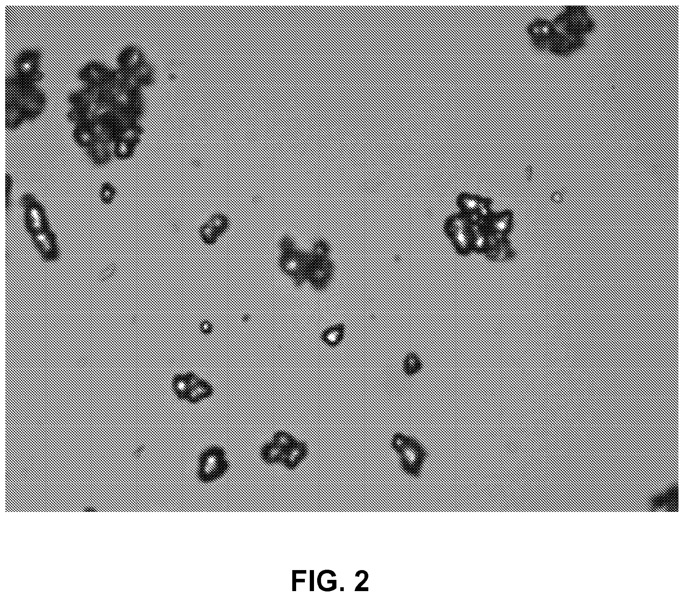

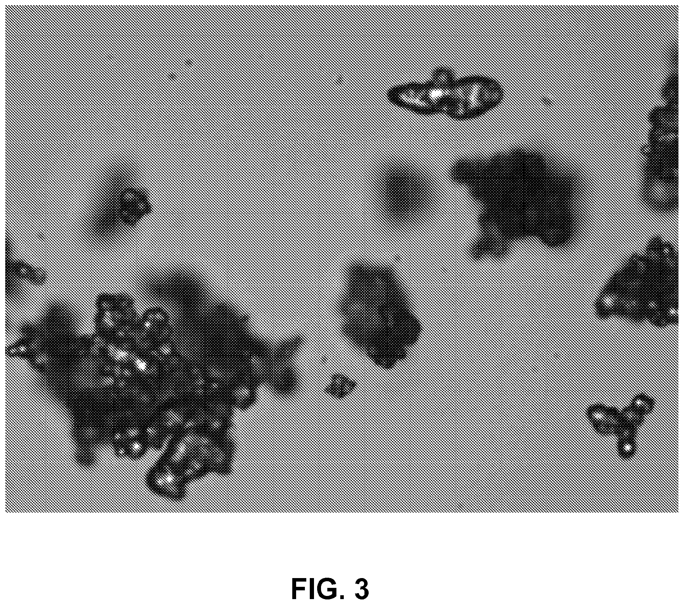

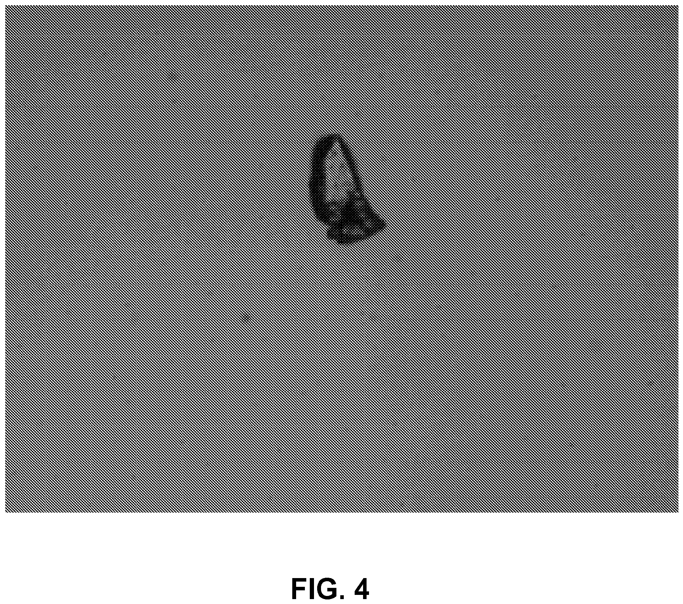

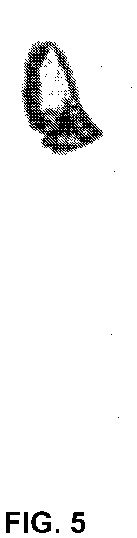

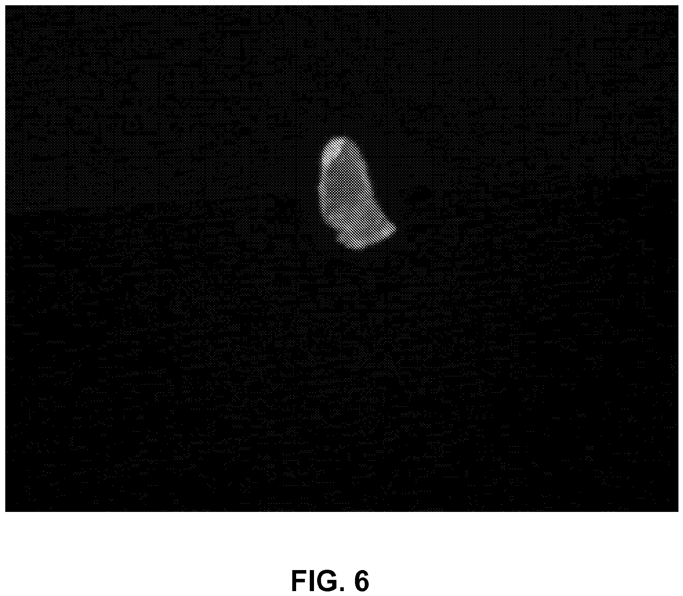

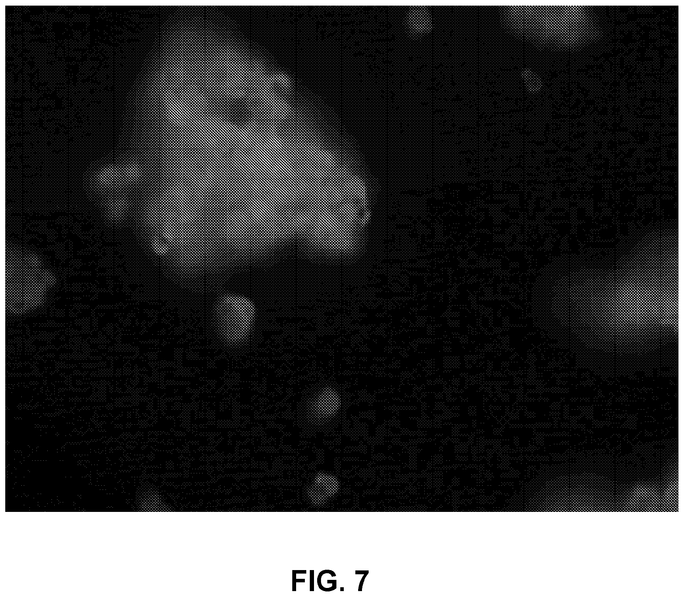

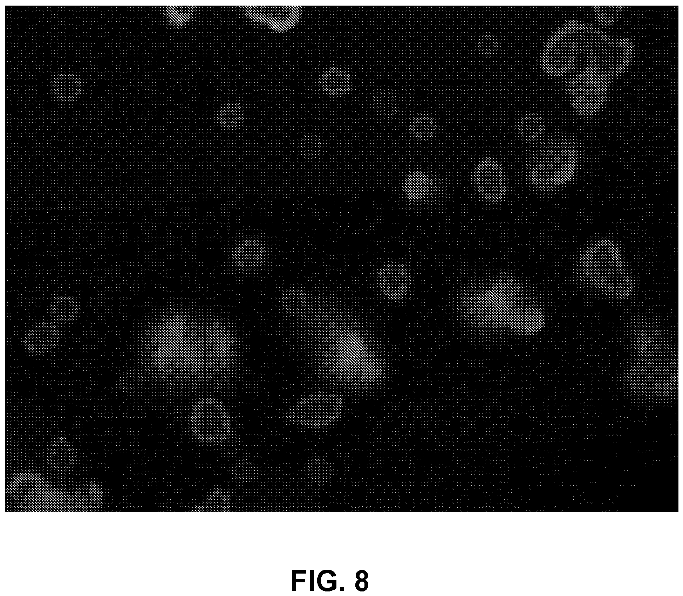

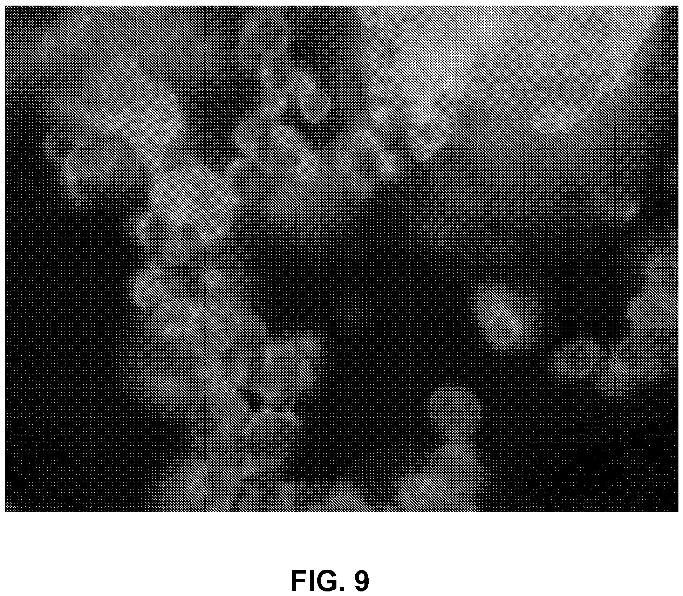

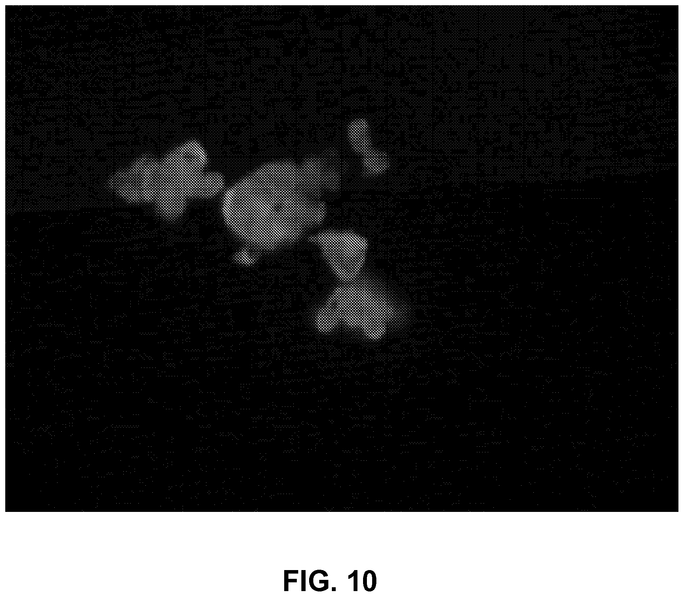

Images of the recrystallized Latanoprost-L-Arginine salt **2** were taken using a compound microscope and camera and are presented in Figures 1-10. The images in Figures 4-6 were taken at 40x magnification, and the images in Figures 1-3 and 7-10 were taken at 400x magnification. The crystals became more spherical over time, which may have indicated the uptake of water.

## Claims

1. An amino acid prostaglandin free acid salt, wherein the amino acid is arginine; and wherein the prostaglandin free acid is selected from the group consisting of latanoprost free acid and travoprost free acid, and wherein the free acid salt is crystalline.

2. A pharmaceutical composition comprising a salt according to claim 1 and a pharmaceutically acceptable carrier.

3. A salt according to claim 1 for use in improving nasal patency, treating glaucoma or other eye disease, skin disorders, respiratory disorders, circulatory disorders, gastrointestinal disorders, hair loss, fertility control, osteoporosis, or neurogenic bladder.

4. A salt according to claim 1 for use in reducing ocular pressure.

## Patentansprüche

1. Aminosäure-freie Prostaglandinsäure-Salz, wobei die Aminosäure Arginin ist; und wobei die freie Prostaglandinsäure ausgewählt ist aus der Gruppe, bestehend aus freier Latanoprostsäure und freier Travoprostsäure, und wobei das freie Säure-Salz kristallin ist.

2. Pharmazeutische Zusammensetzung, umfassend ein Salz nach Anspruch 1 und einen pharmazeutisch verträglichen Träger.

3. Salz nach Anspruch 1 zur Verwendung bei der Verbesserung der Durchgängigkeit der Nase, der Behandlung von Glaukom oder einer anderen Augenkrankheit, Hauterkrankungen, Atemwegserkrankungen, Durchblutungsstörungen, gastrointestinalen Störungen, Haarausfall, Fertilitätskontrolle, Osteoporose oder neurogener Blase.

4. Salz nach Anspruch 1 zur Verwendung bei der Verringerung des Augendrucks.

## Revendications

1. Sel d'acide libre d'acide aminé de prostaglandine, où l'acide aminé est l'arginine ; et l'acide libre de prostaglandine est sélectionné dans le groupe constitué de l'acide libre de latanoprost et de l'acide libre de travoprost, et le sel d'acide libre est cristallin.

2. Composition pharmaceutique comprenant un sel selon la revendication 1 et un support pharmaceutiquement acceptable.

3. Sel selon la revendication 1 pour l'utilisation dans l'amélioration de la patence nasale, le traitement du glaucome ou d'une autre maladie oculaire, des troubles de la peau, des troubles respiratoires, des troubles circulatoires, des troubles gastro-intestinaux, de la perte de cheveux, de la régulation de la fertilité, de l'ostéoporose, ou de la vessie neurogène.

4. Sel selon la revendication 1 pour l'utilisation dans la réduction de la pression oculaire.
